# EUROPEAN PATENT SPECIFICATION

(11) **EP 2 529 227 B1**
(45) Date of publication and mention of the grant of the patent: **07.10.2015**
(21) Application number: 10844862.2
(22) Date of filing: 27.01.2010
(51) Int. Cl.: G01N 33/569, C07K 14/045, G01N 33/574, G01N 33/15

(54) **CYTOMEGALOVIRUS-BASED IMMUNOGENIC PREPARATIONS**
AUF ZYTOMEGALOVIRUS BASIERENDE IMMUNOGENE ZUBEREITUNGEN
PRÉPARATIONS IMMUNOGÈNES À BASE DE CYTOMÉGALOVIRUS

(43) Date of publication of application: 05.12.2012
(73) Proprietor: Oregon Health & Science University, Portland, OR 97201 (US)
(72) Inventor: HILL, Ann, B., Portland OR 97201-3098 (US); SNYDER, Christopher, M., Portland OR 97239 (US)
(74) Representative: Walton, Seán Malcolm
(86) International application number: PCT/US2010/022275
(87) International publication number: WO 2011/093858

(56) References cited:
- US-A- 4 965 069
- US-A- 6 019 978
- US-A1- 2006 240 039
- US-B2- 7 025 968

## Description

### FIELD

This disclosure relates to methods of using recombinant replication-deficient cytomegalovirus (CMV) to generate a long-term, perpetually activated T cell-based immune response in a subject, for instance against a heterologous antigen expressed by the cytomegalovirus. It further relates to methods of using a recombinant replication deficient CMV as an anti-cancer immunogenic preparation.

### BACKGROUND

While there are clear parallels between tumor and viral immunology, the fields are more marked by their differences. Viruses are usually powerfully immunogenic, activate innate immune "danger" signals (*e*.*g.*, Toll-like receptor signaling), and present abundant foreign antigen. Tumors usually contain at best mutated or overexpressed self proteins as antigens, and may provoke immune tolerance rather than immune activation. Thus, one goal of a tumor vaccine is to persuade the immune system to treat a tumor as though it were a virus rather than harmless self tissue. Producing a robust and long-lasting immune response is particularly desirable in light of the finding that a tumor can persist as micrometastases or cancer stem cells after apparent tumor eradication. These cells may themselves divide only rarely, and hence survive radio and chemotherapy. However, their progeny are the typical cancer cells with lethal tumor-producing capability. Since cancer stem cells can remain quiescent for months or years, another goal of tumor vaccination is to provide vigilant immune surveillance long after elimination of the initial tumor burden. Ideally, such immune surveillance would involve CD8⁺ T cells that could patrol tissues to detect micrometastases rather than waiting for tumor to be delivered to draining lymph nodes.

Successful tumor immunotherapy is hampered by the poor immunogenicity of tumor epitopes and the fact that the immune system often responds to them by immune tolerance rather than by initiating a potent effector response. An ideal immunotherapeutic tumor vaccine would elicit an immune response that is (a) robust, (b) effective, (c) resistant to the development of immune tolerance, (d) long-lived, and (e) after initial tumor control, effective at the task of "immune surveillance" against recurrences and metastases. The robust, life-long immune response elicited by cytomegalovirus (CMV) infection, and the ability of CMV to overcome self-tolerance, suggest that CMV may be an ideal vaccine vector for tumor immunotherapy.

CMV is a member of the beta subclass of the herpesvirus family. It is a large (containing a 230 kilobase genome), double stranded DNA virus that establishes life-long latent or persistent infection. In developed countries such as the United States, approximately 70% of the population is infected by CMV. In contrast to gamma herpesviruses such as Epstein-Barr Virus and Kaposi's Sarcoma-associated Herpesvirus, CMV is non-transforming and non-oncogenic. A live, attenuated CMV vaccine (based on the human CMV Towne strain, which lacks a portion of the CMV genome) has been administered by subcutaneous injection to over 800 subjects in a phase II and III safety and efficacy trials (Arvin et al., Clin. Infect. Dis. 39:233-239, 2004). While this vaccine was found to be completely safe, it was not completely efficacious. More recently, in an attempt to increase its efficacy, some of the missing genes in the Towne-based vaccine strain were replaced. This vaccine has been tested in phase II safety studies, and was found to be safe (Arvin et al., Clin. Infect. Dis. 39:233-239, 2004).

The ability of live, recombinant CMV to generate immune responses against recombinant antigens has been demonstrated in several reports (Hansen et al., Nat. Med. 15:293-299, 2009; Karrer et al., J. Virol. 78:2255-2264, 2004). Moreover, it has recently been demonstrated that a recombinant, replication-competent CMV that is engineered to express a self protein will generate long-lasting, CD8⁺ T cell-based immunity against cells expressing the self protein (Lloyd et al., Biol. Reprod. 68:2024-2032, 2003). Since most tumor antigens are over-expressed self proteins, this result provides support for the concept of using a CMV vector to induce an immune response to tumors.

Most notably, Hanson *et al.* used recombinant rhesus CMV expressing SIV antigens to immunize rhesus macaques against SIV (Hansen et al., Nat. Med. 15:293-299, 2009). The immunization induced large numbers of activated "effector memory" CD8⁺ T cells specific for SIV in peripheral tissues, which persisted for the entire multi-year duration of the study. Significantly, the immunized monkeys were substantially protected from SIV challenge, which was attributed to the presence of activated effector-memory T cells. The study also demonstrated that pre-existing immunity to CMV did not prevent the ability of recombinant CMV to induce a new immune response.

Despite the apparent safety of live, attenuated CMV vaccines, significant concerns remain with live CMV-based vaccine strategies. Although in healthy individuals CMV infection is usually completely asymptomatic, problems can arise in immunosuppressed individuals, such as AIDS patients, organ transplant recipients, or infants who were infected *in utero.* Moreover, potential recipients of a CMV-based tumor vaccine may be or become immunodeficient, significantly limiting the utility of a live CMV tumor vaccine. Thus, a continuing need exists for CMV vaccine vector that would be completely safe in immunocompromised individuals.

### SUMMARY

Disclosed herein is the surprising finding that replication-deficient CMV that is completely incapable of spreading between cells is able to produce a long term immune response in a subject. Thus, described herein is a method of generating a long term, repeatedly stimulated immune response against a heterologous cancer antigen in a subject. The method comprises administering to the subject (*e*.*g*., in a single dose), by intraperitoneal or intravenous administration, a recombinant, replication-deficient cytomegalovirus comprising a heterologous nucleic acid encoding the cancer antigen, whereby viral latency is established in the subject, which latency results in the repeatedly stimulated immune response against the antigen.

Also described is a method of treating a subject who has been diagnosed with a cancer comprising administering to the subject one or a combination of a chemotherapeutic or a immunologic anti-cancer agent (s); and administering to the same subject, by e.g. intraperitoneal or intravenous administration, a recombinant, replication-deficient cytomegalovirus comprising a heterologous nucleic acid encoding a heterologous antigen derived from the cancer; whereby viral latency is established in the subject, which latency results in stimulated immunity against the cancer.

The foregoing and other objects, features, and advantages of the invention will become more apparent from the following detailed description, which proceeds with reference to the accompanying figures.

### BRIEF DESCRIPTION OF THE FIGURES

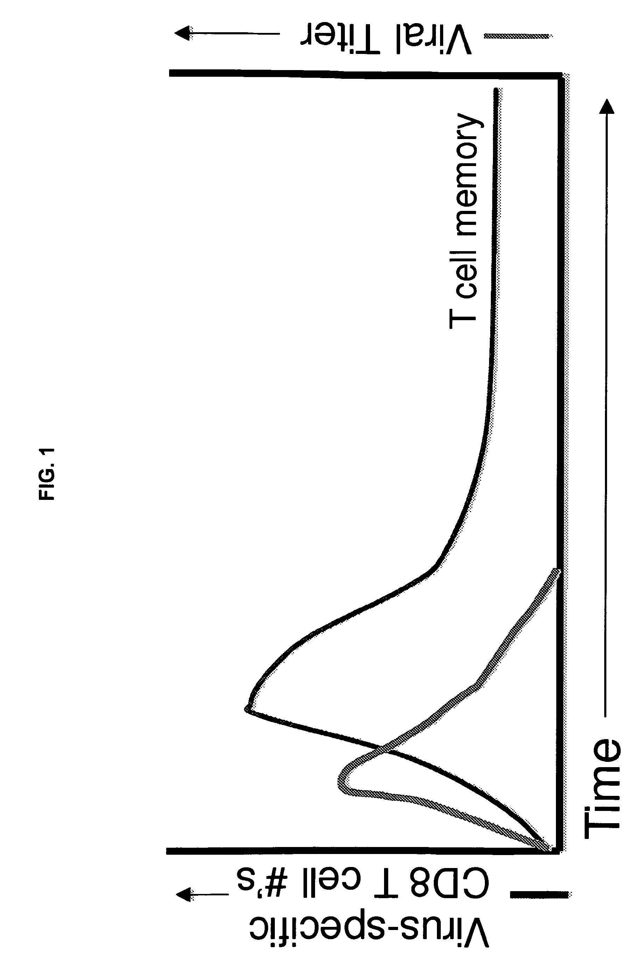

**Figure 1** is a graph comparing virus titer and CMV-reactive T cells over time, following CMV infection.
**Figure 2** shows the immune response elicited by MCMV-IE2-ova vaccination and B16-D5-ova tumor challenge. A) Intracellular cytokine staining (ICS) assay measurement of ova peptide SIINFEKL was measured directly ex vivo in peripheral blood six months after vaccination with MCMV-ova or vector only. Top panels: stimulated with SIINFEKL. Bottom panels: no peptide. B) Measurement of SIINFEKL-specific responses in peripheral blood by ICS, measured as in A, as % of total CD8⁺ T cells in six vaccinated mice, six months post vaccination, but before tumor challenge, and 17 days post tumor challenge. C) Course of tumor progression in vaccinated and control mice shown as percentabe of mice that remained tumor free for the indicated number of days post inoculation. Most animals died within seven days of developing palpable tumor. One vaccinated mouse (#4 from B) remained tumor free.

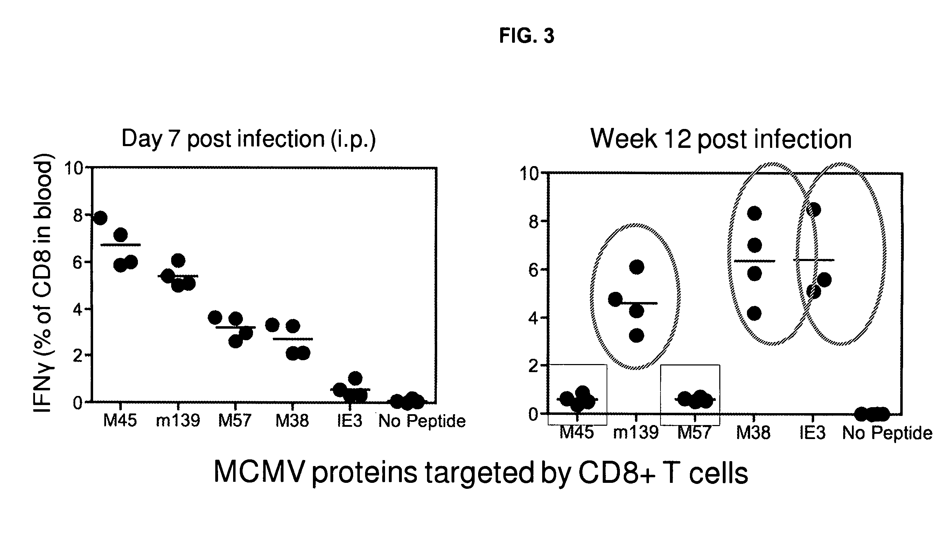

**Figure 3** is a pair of graphs showing the T cell response in MCMV-infected B6 mice. Infection of C57BL/6 mice induces a reproducible immunodominance hierarchy during acute (seven days post) and chronic (seven days post) infection.

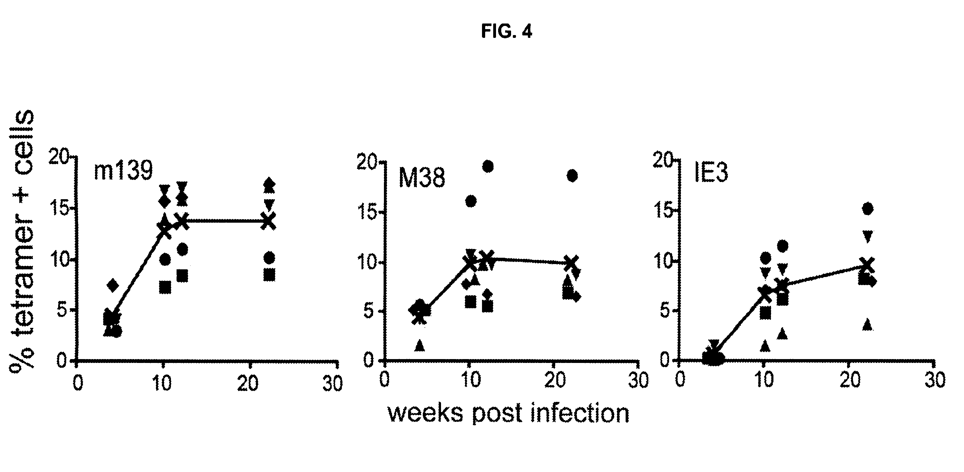

**Figure 4** is a series of graphs showing the memory inflation of CD8⁺ T cells in mice infected with replication-competent MCMV. C57BL/6 mice (n-5) were infected with MCMV at week 0. At the indicated weeks post infection, peripheral blood was obtained by tail bleed and response to three MCMV epitopes (m139, m38 and IE3) measured by tetramer staining. Epitope-specific responses are graphed as a percentage of total CD8⁺ lymphocytes in the blood. Symbols indicate individual animals; the line indicates the mean. Background tetramer staining on uninfected controls was <0.2%.

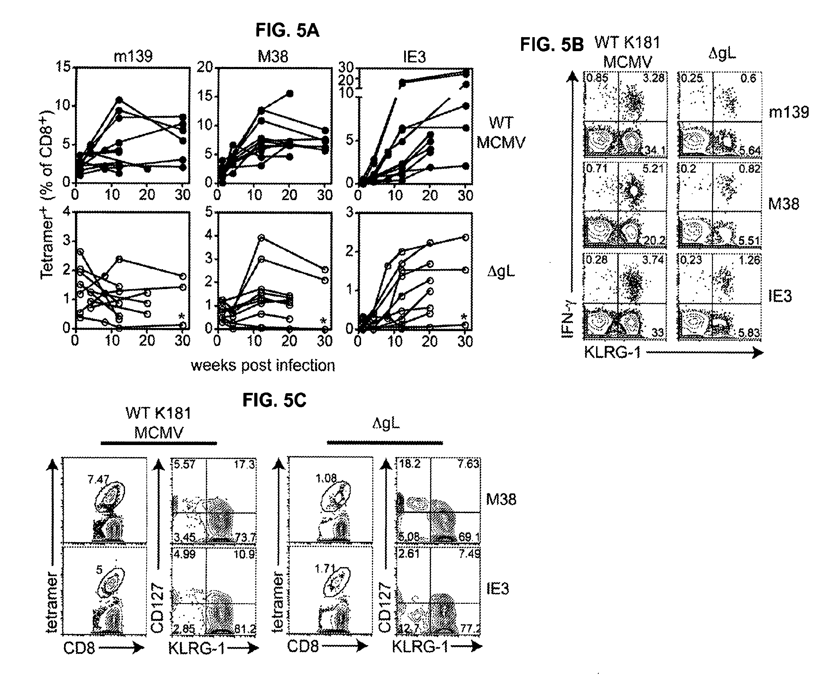

**Figure 5** is a series of graphs showing memory inflation of CD8⁺ T cells in mice infected with replication-deficient MCMV. A) Mice infected with wildtype MCMV are shown in filled circles and mice infected with replication-deficient, ΔgL MCMV, are shown in open circles. Each line represents an individual mouse analyzed at the indicated time points. Data is graphed as a % of total CD8⁺ T cells in the peripheral blood as assessed by tetramer staining. B) Representative FACS profiles of CD8⁺ T cells from wildtype and ΔgL infected mice after stimulation of T cells with the indicated peptides. T cells were assessed for IFN-γ production (Y-axis) and KLRG-1 expression (X-axis) 25 weeks post infection. C) Representative FACS profiles of antigen-specific cells from wildtype and ΔgL infected mice 20 weeks after infection showing CD8 expression (X-axis) and tetramer staining (Y-axis) to indicate how tetramer-binding T cells were gated. Additional FACS profiles show KLRG-1 expression (X-axis) and CD127 expression (IL-7Rα, Y-axis) after gating on tetramer positive T cells.

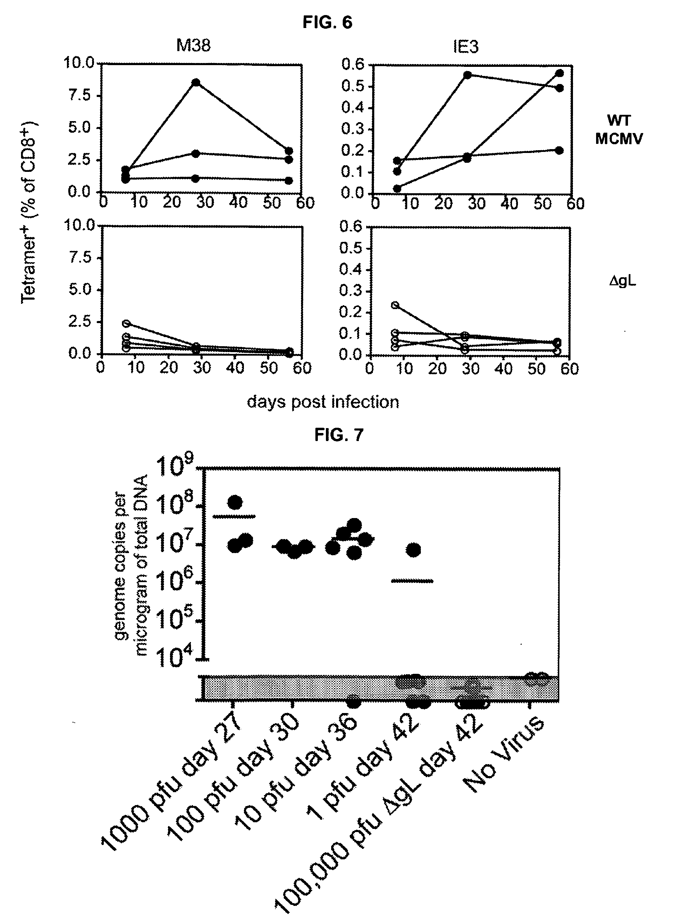

**Figure 6** is a series of graphs showing the immune response elicited by wild-type MCMV or ΔgL replication deficient MCMV after subcutaneous infection in the foot pad. T cell responses were measured over time in the peripheral blood by tetramer staining as in Figure 5A. Filled circles represent wildtype infected mice. Open circles represent ΔgL infected mice. Each line represents an individual animal tested at the indicated times post infection.
**Figure 7** is a graph showing the number of viral genomes present in the spleens of severe combined immunodeficient (SCID) mice infected with wild-type or ΔgL MCMV at various times post infection. Balb/c-SCID mice were infected at day 0 with varying amounts of wild-type virus or 1x10⁵ pfu of ΔgL virus. Animals within each group were sacrificed when any member of that group exhibited signs of illness. Mice infected with ΔgL virus never exhibited signs of illness for six weeks after infection.

### SEQUENCE LISTING

The nucleic and/or amino acid sequences listed in the accompanying sequence listing are shown using standard letter abbreviations for nucleotide bases, and three letter code for amino acids, as defined in 37 C.F.R. 1.822. Only one strand of each nucleic acid sequence is shown, but the complementary strand is understood as included by any reference to the displayed strand. In the accompanying sequence listing:
SEQ ID NO: 1 is the nucleic acid sequence of a representative probe for MCMV E1.
SEQ ID NOs: 2 and 3 are the nucleic acid sequences for representative forward and reverse (respectively) primers for MCMV E1.

### DETAILED DESCRIPTION

### I. Abbreviations

- **cDNA**: complementary DNA
- **CMV**: cytomegalovirus
- **CTL**: cytotoxic T lymphocyte
- **DC**: dendritic cell
- **EF-1a**: elongation factor 1 alpha
- **FACS**: fluorescence activated cell sorting
- **GP**: glycoprotein
- **HSC**: hematopoietic stem cell
- **ICS**: intracellular cytokine staining
- **IE**: immediate early
- **IV**: intravenous
- **IP**: intraperitoneal
- **KB**: kilobase
- **PFU**: plaque forming unit
- **SCID**: severe combined immunodeficient
- **TAA**: tumor associated antigen

### II. Terms

Unless otherwise noted, technical terms are used according to conventional usage. Definitions of common terms in molecular biology may be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

In order to facilitate review of the various embodiments of the invention, the following explanations of specific terms are provided:

**Administration**: Administration of an active compound or composition can be by any route known to one of skill in the art. Administration can be local or systemic. Examples of local administration include, but are not limited to, topical administration, subcutaneous administration, intramuscular administration, intrathecal administration, intrapericardial administration, intra-ocular administration, topical ophthalmic administration, or administration to the nasal mucosa or lungs by inhalational administration. In addition, local administration includes routes of administration typically used for systemic administration such as intravenous and intraperitoneal routes, for example by directing intravascular administration to the arterial supply for a particular organ. Thus, in particular embodiments, local administration includes intra-arterial administration and intravenous administration when such administration is targeted to the vasculature supplying a particular organ. Local administration also includes the incorporation of active compounds and agents into implantable devices or constructs, such as vascular stents or other reservoirs, which release the active agents and compounds over extended time intervals for sustained treatment effects.

Systemic administration includes any route of administration designed to distribute an active compound or composition widely throughout the body via the circulatory system. Thus, systemic administration includes, but is not limited to intraperitoneal, intra-arterial and intravenous administration. Systemic administration also includes, but is not limited to, topical administration, subcutaneous administration, intramuscular administration, or administration by inhalation, when such administration is directed at absorption and distribution throughout the body by the circulatory system.

**Animal**: A living multi-cellular vertebrate or invertebrate organism, a category that includes, for example, mammals and birds. The term mammal includes both human and non-human mammals. The term "**primate**" includes both human and non-human primates. "**Non-human primates**" are simian primates such as monkeys, chimpanzees, orangutans, baboons, and macaques. Similarly, the term "**subject**" includes both human and veterinary subjects, such as non-human primates.

**Antibody**: Immunoglobulin molecules and immunologically active portions of immunoglobulin molecules, for instance, molecules that contain an antigen binding site that specifically binds (immunoreacts with) an antigen.

A naturally occurring antibody (for example, IgG, IgM, IgD) includes four polypeptide chains, two heavy (H) chains and two light (L) chains inter-connected by disulfide bonds. However, it has been shown that the antigen-binding function of an antibody can be performed by fragments of a naturally occurring antibody. Thus, these antigen-binding fragments are also intended to be designated by the term "antibody." Specific, non-limiting examples of binding fragments encompassed within the term antibody include (i) an Fab fragment consisting of the V_{L}, V_{H}, CL, and CH1 domains; (ii) an Fd fragment consisting of the V_{H} and CH1 domains; (iii) an Fv fragment consisting of the V_{L} and V_{H} domains of a single arm of an antibody, (iv) a dAb fragment (Ward et al., Nature 341:544-546, 1989) which consists of a V_{H} domain; (v) an isolated complementarity determining region (CDR); and (vi) an F(ab')₂ fragment, a bivalent fragment comprising two Fab fragments linked by a disulfide bridge at the hinge region.

Immunoglobulins and certain variants thereof are known and many have been prepared in recombinant cell culture (for example, see U.S. Patent No. 4,745,055; U.S. Patent No. 4,444,487; WO 88/03565; EP 0256654; EP 0120694; EP 0125023; Faoulkner et al., Nature 298:286, 1982; Morrison, J. Immunol. 123:793, 1979; Morrison et al., Ann Rev. Immunol 2:239, 1984).

**Antigen**: A substance that can stimulate the production of antibodies or a T cell response in a mammal, including compositions that are injected or absorbed into a mammal. An antigen reacts with the products of specific humoral or cellular immunity, including those induced by heterologous immunogens. The term "antigen" includes all related antigenic epitopes. In the invention, the antigen is a cancer antigen. **A target antigen** is an antigen against which an immune response is desired, for example to achieve a therapeutic effect, such as tumor regression.

**Antigen-speciric T cell:** A CD8⁺ or CD4⁺ lymphocyte that recognizes a particular antigen. Generally, antigen-specific T cells specifically bind to a particular antigen presented by MHC molecules, but not other antigens presented by the same MHC.

**Cancer**: Malignant neoplasm that has undergone characteristic anaplasia with loss of differentiation, increased rate of growth, invasion of surrounding tissue, and is capable of metastasis.

**cDNA (complementary DNA):** A piece of DNA lacking internal, noncoding segments (introns) and transcriptional regulatory sequences. cDNA may also contain untranslated regions (UTRs) that are responsible for translational control in the corresponding RNA molecule. cDNA is usually synthesized in the laboratory by reverse transcription from messenger RNA extracted from cells.

**CMV (cytomegalovirus)**: A member of the beta subclass of the family of herpesviruses. CMV is a large (containing a 230 kilobase genome), double stranded DNA virus, with host-range specific variants such as MCMV (murine CMV) and HCMV (human CMV).

**Chemotherapy**: In cancer treatment, chemotherapy refers to the administration of one or more agents to kill or slow the reproduction of rapidly multiplying cells, such as tumor or cancer cells. In a particular example, chemotherapy refers to the administration of one or more anti-neoplastic agents to significantly reduce the number of tumor cells in the subject, such as by at least 50%. Cytotoxic anti-tumor chemotherapeutic agents include, but are not limited to: 5-fluorouracil (5-FU), azathioprine, cyclophosphamide (such as Cytoxan®), antimetabolites (such as Fludarabine), and other antineoplastics such as Etoposide, Doxorubicin, methotrexate, Vincristine, carboplatin, cis-platinum and the taxanes (such as taxol).

**Chemotherapeutic agent:** An agent with therapeutic usefulness in the treatment of diseases characterized by abnormal cell growth or hyperplasia. Such diseases include cancer, autoimmune disease as well as diseases characterized by hyperplastic growth such as psoriasis. One of skill in the art can readily identify a chemotherapeutic agent (for instance, see Slapak and Kufe, Principles of Cancer Therapy, Chapter 86 in Harrison's Principles of Internal Medicine, 14th edition; Perry et al., Chemotherapy, Ch. 17 in Abeloff, Clinical Oncology 2nd ed., © 2000 Churchill Livingstone, Inc; Baltzer L, Berkery R (eds): Oncology Pocket Guide to Chemotherapy, 2nd ed. St. Louis, Mosby-Year Book, 1995; Fischer DS, Knobf MF, Durivage HJ (eds): The Cancer Chemotherapy Handbook, 4th ed. St. Louis, Mosby-Year Book, 1993). Examples of chemotherapeutic agents include ICL-inducing agents, such as melphalan (Alkeran^{™}), cyclophosphamide (Cytoxan^{™}), cisplatin (Platinol^{™}) and busulfan (Busilvex^{™}, Myleran^{™}).

**Decrease or deplete:** To reduce the quality, amount, or strength of something.

In one example, a therapy (such as the methods provided herein) decreases a tumor (such as the size of a tumor, the number of tumors, the metastasis of a tumor, the reoccurrence of a tumor or combinations thereof), or one or more symptoms associated with a tumor, for example as compared to the response in the absence of the therapy. In a particular example, a therapy decreases the size of a tumor, the number of tumors, the metastasis of a tumor, the reoccurrence of a tumor or combinations thereof, subsequent to the therapy, such as a decrease of at least 10%, at least 20%, at least 50%, or even at least 90%.

**DNA (deoxyribonucleic acid):** DNA is a long chain polymer which comprises the genetic material of most living organisms (some viruses have genes comprising ribonucleic acid (RNA)). The repeating units in DNA polymers are four different nucleotides, each of which comprises one of the four bases, adenine (A), guanine (G), cytosine (C), and thymine (T) bound to a deoxyribose sugar to which a phosphate group is attached.

Unless otherwise specified, any reference to a DNA molecule is intended to include the complementary sequence of that DNA molecule. Except where single-strandedness is required by the text herein, DNA molecules, though written to depict only a single strand, encompass both strands of a double-stranded DNA molecule. Thus, a reference to the nucleic acid molecule that encodes a specific protein, or a fragment thereof, encompasses both the sense strand and its complementary strand. For instance, it is appropriate to generate probes or primers from the complementary sequence of the disclosed nucleic acid molecules.

**Deletion**: The removal of a sequence of DNA, the regions on either side of the removed sequence being joined together.

**Effective amount:** A quantity sufficient to achieve a desired effect in a subject being treated. An effective amount of a composition, such as a vaccine, can be administered in a single dose, or in several doses, during a course of treatment. However, the effective amount of the compound will be dependent on the compound applied, the subject being treated, the severity and type of the affliction, and the manner of administration of the compound.

**Encode:** A polynucleotide is said to encode a polypeptide if, in its native state or when manipulated by methods well known to those skilled in the art, it can be transcribed and/or translated to produce the mRNA for and/or the polypeptide or a fragment thereof.

**Epitope:** An antigenic determinant. These are particular chemical groups or peptide sequences on a molecule that are antigenic, such that they elicit a specific immune response. For example, an epitope may be a tumor-derived polypeptide that is expressed from a nucleic acid delivered to a cell by a recombinant replication deficient CMV vaccine. Antibodies that are generated as part of the immune response elicited by this vaccine will bind the particular antigenic tumor-derived epitope.

**Expression:** Translation of a nucleic acid into a protein, for example the translation of a mRNA encoding a tumor antigen into a protein.

**Expression Control Sequences:** Nucleic acid sequences that regulate the expression of a heterologous nucleic acid sequence to which it is operatively linked, for example the expression of a heterologous polynucleotide spliced in a CMV genome and encoding an antigenic protein operably linked to expression control sequences. Expression control sequences are operatively linked to a nucleic acid sequence when the expression control sequences control and regulate the transcription and, as appropriate, translation of the nucleic acid sequence. Thus expression control sequences can include appropriate promoters, enhancers, transcription terminators, a start codon (ATG) in front of a protein-encoding gene, splicing signal for introns, maintenance of the correct reading frame of that gene to permit proper translation of mRNA, and stop codons. The term "control sequences" is intended to include, at a minimum, components whose presence can influence expression, and can also include additional components whose presence is advantageous, for example, leader sequences and fusion partner sequences. Expression control sequences can include a promoter.

A promoter is a minimal sequence sufficient to direct transcription. Also included are those promoter elements which are sufficient to render promoter-dependent gene expression controllable for cell-type specific, tissue-specific, or inducible by external signals or agents; such elements may be located in the 5' or 3' regions of the gene. Both constitutive and inducible promoters are included (see for example, Bitter et al., Methods in Enzymology 153:516-544, 1987). For example, when cloning in bacterial systems, inducible promoters such as pL of bacteriophage lambda, plac, ptrp, ptac (ptrp-lac hybrid promoter) and the like may be used. In one embodiment, when cloning in mammalian cell systems, promoters derived from the genome of mammalian cells (such as metallothionein promoter) or from mammalian viruses (such as the retrovirus long terminal repeat; the adenovirus late promoter; the vaccinia virus 7.5K promoter) can be used. Promoters produced by recombinant DNA or synthetic techniques may also be used to provide for transcription of the nucleic acid sequences.

A polynucleotide can be inserted into an expression vector, including a viral vector, that contains a promoter sequence, which facilitates the efficient transcription of the inserted genetic sequence of the host. The expression vector typically contains an origin of replication, a promoter, as well as specific nucleic acid sequences that allow phenotypic selection of the transformed cells.

**Gene expression:** The process by which the coded information of a nucleic acid transcriptional unit (including, for example, genomic DNA or cDNA) is converted into an operational, non-operational, or structural part of a cell, often including the synthesis of a protein. Gene expression can be influenced by external signals; for instance, exposure of a subject to an agent that inhibits gene expression. Expression of a gene also may be regulated anywhere in the pathway from DNA to RNA to protein. Regulation of gene expression occurs, for instance, through controls acting on transcription, translation, RNA transport and processing, degradation of intermediary molecules such as mRNA, or through activation, inactivation, compartmentalization or degradation of specific protein molecules after they have been made, or by combinations thereof. Gene expression may be measured at the RNA level or the protein level and by any method known in the art, including Northern blot, RT-PCR, Western blot, or *in vitro,* in situ, or *in vivo* protein activity assay(s).

The expression of a nucleic acid may be modulated compared to a control state, such as at a control time (for example, prior to administration of a substance or agent that affects regulation of the nucleic acid under observation) or in a control cell or subject, or as compared to another nucleic acid. Such modulation includes but is not necessarily limited to overexpression, underexpression, or suppression of expression. In addition, it is understood that modulation of nucleic acid expression may be associated with, and in fact may result in, a modulation in the expression of an encoded protein or even a protein that is not encoded by that nucleic acid.

**Heterologous:** A type of sequence that is not normally (for example, in the wild-type sequence) found adjacent to a second sequence. In one embodiment, the sequence is from a different genetic source, such as a virus or other organism, than the second sequence.

**Hyperproliferative disease:** A disease or disorder characterized by the uncontrolled proliferation of cells. Hyperproliferative diseases include, but are not limited to malignant and non-malignant tumors.

**Immunogenic peptide:** A peptide which comprises an allele-specific motif or other sequence, such as an N-terminal repeat, such that the peptide will bind an MHC molecule and induce a cytotoxic T lymphocyte ("CTL") response, or a B cell response (for example antibody production) against the antigen from which the immunogenic peptide is derived.

In one embodiment, immunogenic peptides are identified using sequence motifs or other methods, such as neural net or polynomial determinations known in the art. Typically, algorithms are used to determine the "binding threshold" of peptides to select those with scores that give them a high probability of binding at a certain affinity and will be immunogenic. The algorithms are based either on the effects on MHC binding of a particular amino acid at a particular position, the effects on antibody binding of a particular amino acid at a particular position, or the effects on binding of a particular substitution in a motif-containing peptide. Within the context of an immunogenic peptide, a "conserved residue" is one which appears in a significantly higher frequency than would be expected by random distribution at a particular position in a peptide. In one embodiment, a conserved residue is one where the MHC structure may provide a contact point with the immunogenic peptide.

**Immunologically reactive conditions:** Includes reference to conditions which allow an antibody raised against an epitope, to bind to that epitope to a detectably greater degree than, and/or to the substantial exclusion of, binding to substantially all other epitopes. Immunologically reactive conditions are dependent upon the format of the antibody binding reaction and typically are those utilized in immunoassay protocols or those conditions encountered *in vivo.* The immunologically reactive conditions employed in the methods are "physiological conditions" which include reference to conditions (such as temperature, osmolarity, pH) that are typical inside a living mammal or a mammalian cell. While it is recognized that some organs are subject to extreme conditions, the intra-organismal and intracellular environment is normally about pH 7 (such as from pH 6.0 to pH 8.0, more typically pH 6.5 to 7.5), contains water as the predominant solvent, and exists at a temperature above 0°C and below 50°C. Osmolarity is within the range that is supportive of cell viability and proliferation.

**Immune response:** A change in immunity, for example a response of a cell of the immune system, such as a B-cell, T cell, macrophage, monocyte, or polymorphonucleocyte, to an immunogenic agent in a subject. The response can be specific for a particular antigen (an "antigen-specific response"). In a particular example, an immune response is a T cell response, such as a CD4⁺ response or a CD8⁺ response. In another example, the response is a B-cell response, and results in the production of specific antibodies to the immunogenic agent.

In some examples, such an immune response provides protection for the subject from the immunogenic agent or the source of the immunogenic agent. For example, the response can treat a subject having a tumor, for example by interfering with the metastasis of the tumor or reducing the number or size of a tumor. An immune response can be active and involve stimulation of the subject's immune system, or be a response that results from passively acquired immunity. A "repeatedly stimulated" immune response is a long-term immune response resulting from the periodic and repetitive stimulation of the immune system by the repeated production of an antigen within a host.

In a particular example, an increased or enhanced immune response is an increase in the ability of a subject to fight off a disease, such as a tumor.

**Immunity:** The state of being able to mount a protective response upon exposure to an immunogenic agent. Protective responses can be antibody-mediated or immune cell-mediated, and can be directed toward a particular pathogen or tumor antigen. Immunity can be acquired actively (such as by exposure to an immunogenic agent, either naturally or in a pharmaceutical composition) or passively (such as by administration of antibodies or in vitro stimulated and expanded T cells).

In a particular example, immunity is acquired by intraperitoneal or intravenous administration of a replication-deficient CMV that is expressing a particular antigen, such as a tumor antigen.

**Immunotherapy:** A method of evoking an immune response against on their production of target antigens. Immunotherapy based on cell-mediated immune responses involves generating a cell-mediated response to cells that produce particular antigenic determinants, while immunotherapy based on humoral immune responses involves generating specific antibodies to virus that produce particular antigenic determinants. In particular non-limiting examples, a cell-mediated immune response is generated by infecting a subjected with a recombinant replication-deficient CMV that is expressing a tumor antigen.

**Infectious disease:** A disease caused by a pathogen, such as a fungus, parasite, bacterium or virus.

**Interferon-gamma (IFN-γ):** A protein produced by T lymphocytes in response to specific antigen or mitogenic stimulation. Includes naturally occurring IFN-γ peptides and nucleic acid molecules and IFN-γ fragments and variants that retain full or partial IFN-γ biological activity. Sequences for IFN-γ are publicly available (for example, exemplary IFN-γ mRNA sequences are available from GenBank Accession Nos: BC070256; AF506749; and J00219, and exemplary IFS-γ protein sequences are available from GenBank Accession Nos: CAA00226; AAA72254; and 0809316A).

Methods of measuring functional IFN-γ are known, and include, but are not limited to: immunoassays. For example, the public availability of antibodies that recognize IFN-γ permits the use of ELISA and flow cytometry to detect cells producing IFN-γ. Another method is a cytotoxicity assay that measures the level of killing of tumor targets by activated T cells (for example see Hu et al., J. Immunother. 27:48-59, 2004, and Walker et al., Clin. Cancer Res. 10:668-80, 2004).

**Inhibiting or treating a disease:** Inhibiting the full development or recurrence of a disease or condition, for example, in a subject who is at risk for or been diagnosed with a cancer such as melanoma. "Treatment" refers to a therapeutic intervention that ameliorates a sign or symptom of a disease or pathological condition after it has begun to develop. The term "ameliorating," with reference to a disease or pathological condition, refers to any observable beneficial effect of the treatment. The beneficial effect can be evidenced, for example, by a delayed onset or recurrence of clinical symptoms of the disease in a susceptible subject, a reduction in severity of some or all clinical symptoms of the disease, a slower progression of the disease, a reduction in the number of metastases, an improvement in the overall health or well-being of the subject, or by other parameters well known in the art that are specific to the particular disease. A "prophylactic" treatment is a treatment administered to a subject who does not exhibit signs of a disease or exhibits only early signs for the purpose of decreasing the risk of developing pathology.

**Isolated:** An "isolated" biological component (such as a nucleic acid molecule, protein or organelle) has been substantially separated or purified away from other biological components in the cell of the organism in which the component naturally occurs, e.g., other chromosomal and extra-chromosomal DNA and RNA, proteins and organelles. Nucleic acids and proteins that have been "isolated" include nucleic acids and proteins purified by standard purification methods. The term also embraces nucleic acids and proteins prepared by recombinant expression in a host cell as well as chemically synthesized nucleic acids.

**Mutation:** Any change of DNA sequence, for instance within a gene or chromosome. In some instances, a mutation will alter a characteristic or trait (phenotype), but this is not always the case. Types of mutations include base substitution point mutations (for example, transitions or transversions), deletions, and insertions. Missense mutations are those that introduce a different amino acid into the sequence of the encoded protein; nonsense mutations are those that introduce a new stop codon. In the case of insertions or deletions, mutations can be in-frame (not changing the frame of the overall sequence) or frame shift mutations, which may result in the misreading of a large number of codons (and often leads to abnormal termination of the encoded product due to the presence of a stop codon in the alternative frame). One specific deletion mutation is a so-called "knockout mutation," which removes essentially all of the coding sequence of a gene or otherwise renders it incapable of serving as a template for production of a message transcript.

The term mutation specifically encompasses variations that arise through somatic mutation, for instance those that are found only in disease cells, but not constitutionally, in a given individual. Examples of such somatically-acquired variations include the point mutations that frequently result in altered function of various genes that are involved in development of cancers. This term also encompasses DNA alterations that are present constitutionally, that alter the function of the encoded protein in a readily demonstrable manner, and that can be inherited by the children of an affected individual. In this respect, the term overlaps with polymorphism, but generally refers to the subset of constitutional alterations that have arisen within the past few generations in kindred and that are not widely disseminated in a population group.

In particular examples, a mutation is inactivating, whereby the function of the native gene is completely disrupted.

**Nucleic acid molecule:** A polymeric form of nucleotides, which may include both sense and anti-sense strands of RNA, cDNA, genomic DNA, and synthetic forms and mixed polymers thereof. A nucleotide refers to a ribonucleotide, deoxynucleotide or a modified form of either type of nucleotide. A nucleic acid molecule as used herein is synonymous with nucleic acid and polynucleotide. A nucleic acid molecule is usually at least 10 bases in length, unless otherwise specified. The term includes single- and double-stranded forms. A polynucleotide may include either or both naturally occurring and modified nucleotides linked together by naturally occurring nucleotide linkages and/or non-naturally occurring chemical linkers.

Nucleic acid molecules may be modified chemically or biochemically or may contain non-natural or derivatized nucleotide bases, as will be readily appreciated by those of skill in the art. Such modifications include, for example, labels, methylation, substitution of one or more of the naturally occurring nucleotides with an analog, internucleotide modifications, such as uncharged linkages (for example, methyl phosphonates, phosphotriesters, phosphoramidates, carbamates, etc.), charged linkages (for example, phosphorothioates, phosphorodithioates, etc.), pendent moieties (for example, polypeptides), intercalators (for example, acridine, psoralen, etc.), chelators, alkylators, and modified linkages (for example, alpha anomeric nucleic acids, etc.). The term nucleic acid molecule also includes any topological conformation, including single-stranded, double-stranded, partially duplexed, triplexed, hairpinned, circular and padlocked conformations. Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule.

Unless specified otherwise, the left hand end of a polynucleotide sequence written in the sense orientation is the 5' end and the right hand end of the sequence is the 3' end. In addition, the left hand direction of a polynucleotide sequence written in the sense orientation is referred to as the 5' direction, while the right hand direction of the polynucleotide sequence is referred to as the 3' direction. Further, unless otherwise indicated, each nucleotide sequence is set forth herein as a sequence of deoxyribonucleotides. It is intended, however, that the given sequence be interpreted as would be appropriate to the polynucleotide composition: for example, if the isolated nucleic acid is composed of RNA, the given sequence intends ribonucleotides, with uridine substituted for thymidine.

An anti-sense nucleic acid is a nucleic acid (such as, an RNA or DNA oligonucleotide) that has a sequence complementary to a second nucleic acid molecule (for example, an mRNA molecule). An anti-sense nucleic acid will specifically bind with high affinity to the second nucleic acid sequence. If the second nucleic acid sequence is an mRNA molecule, for example, the specific binding of an anti-sense nucleic acid to the mRNA molecule can prevent or reduce translation of the mRNA into the encoded protein or decrease the half life of the mRNA, and thereby inhibit the expression of the encoded protein.

**Nucleotide:** "Nucleotide" includes, but is not limited to, a monomer that includes a base linked to a sugar, such as a pyrimidine, purine or synthetic analogs thereof, or a base linked to an amino acid, as in a peptide nucleic acid (PNA). A nucleotide is one monomer in a polynucleotide. A nucleotide sequence refers to the sequence of bases in a polynucleotide.

**Oligonucleotide:** An oligonucleotide is a plurality of joined nucleotides joined by native phosphodiester bonds, between about 6 and about 300 nucleotides in length. An oligonucleotide analog refers to moieties that function similarly to oligonucleotides but have non-naturally occurring portions. For example, oligonucleotide analogs can contain non-naturally occurring portions, such as altered sugar moieties or inter-sugar linkages, such as a phosphorothioate oligodeoxynucleotide. Functional analogs of naturally occurring polynucleotides can bind to RNA or DNA, and include peptide nucleic acid (PNA) molecules.

Particular oligonucleotides and oligonucleotide analogs can include linear sequences up to about 200 nucleotides in length, for example a sequence (such as DNA or RNA) that is at least 6 bases, for example at least 8, 10, 15, 20, 25, 30, 35, 40, 45, 50, 100 or even 200 bases long, or from about 6 to about 50 bases, for example about 10-25 bases, such as 12, 15 or 20 bases.

**Operably linked:** A first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein-coding regions, in the same reading frame.

**Open reading frame:** A series of nucleotide triplets (codons) coding for amino acids without any internal termination codons. These sequences are usually translatable into a peptide.

**Pharmaceutically acceptable carriers:** The pharmaceutically acceptable carriers useful with this disclosure are conventional. Martin, Remington's Pharmaceutical Sciences, published by Mack Publishing Co., Easton, PA, 19th Edition, 1995, describes compositions and formulations suitable for pharmaceutical delivery of the nucleotides and proteins herein disclosed.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions *(e.g.,* powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Plaque forming units (pfu):** A measure of virus dose, determined by its ability to form plaques on a permissive, complementing cell line.

**Polypeptide:** A polymer in which the monomers are amino acid residues that are joined together through amide bonds. When the amino acids are alpha-amino acids, either the L-optical isomer or the D-optical isomer can be used, the L-isomers being preferred. The term polypeptide or protein as used herein encompasses any amino acid sequence and includes modified sequences such as glycoproteins. The term polypeptide is specifically intended to cover naturally occurring proteins, as well as those that are recombinantly or synthetically produced.

The term polypeptide fragment refers to a portion of a polypeptide that exhibits at least one useful epitope. The phrase "functional fragment(s) of a polypeptide" refers to all fragments of a polypeptide that retain an activity, or a measurable portion of an activity, of the polypeptide from which the fragment is derived. Fragments, for example, can vary in size from a polypeptide fragment as small as an epitope capable of binding an antibody molecule to a large polypeptide capable of participating in the characteristic induction or programming of phenotypic changes within a cell. An epitope is a region of a polypeptide capable of binding an immunoglobulin generated in response to contact with an antigen. Thus, smaller peptides containing the biological activity of insulin, or conservative variants of the insulin, are thus included as being of use.

Conservative amino acid substitution tables providing functionally similar amino acids are well known to one of ordinary skill in the art. The following six groups are examples of amino acids that are considered to be conservative substitutions for one another:
1) Alanine (A), Serine (S), Threonine (T);
2) Aspartic acid (D), Glutamic acid (E);
3) Asparagine (N), Glutamine (Q);
4) Arginine I, Lysine (K);
5) Isoleucine (I), Leucine (L), Methionine (M), Valine (V); and
6) Phenylalanine (F), Tyrosine (Y), Tryptophan (W).

In some circumstances, variations in the cDNA sequence that result in amino acid changes, whether conservative or not, are minimized in order to preserve the functional and immunologic identity of the encoded protein. The immunologic identity of the protein may be assessed by determining whether it is recognized by an antibody; a variant that is recognized by such an antibody is immunologically conserved. Any cDNA sequence variant will preferably introduce no more than twenty, and preferably fewer than ten amino acid substitutions into the encoded polypeptide. Variant amino acid sequences may, for example, be 80%, 90%, or even 95% or 98% identical to the native amino acid sequence. Programs and algorithms for determining percentage identity can be found at the NCBI website.

**Protein:** A biological molecule, particularly a polypeptide, expressed by a gene and comprised of amino acids.

**Purified:** The term "purified" does not require absolute purity; rather, it is intended as a relative term. Thus, for example, a purified protein preparation is one in which the protein referred to is more pure than the protein in its natural environment within a cell or within a production reaction chamber (as appropriate).

**Recombinant:** A recombinant nucleic acid is one that has a sequence that is not naturally occurring or has a sequence that is made by an artificial combination of two otherwise separated segments of sequence. This artificial combination can be accomplished by chemical synthesis or, more commonly, by the artificial manipulation of isolated segments of nucleic acids, *e.g.,* by genetic engineering techniques.

**Sequence identity:** The similarity between two nucleic acid sequences, or two amino acid sequences, is expressed in terms of the similarity between the sequences, otherwise referred to as sequence identity. Sequence identity is frequently measured in terms of percentage identity (or similarity or homology); the higher the percentage, the more similar the two sequences are.

Methods of alignment of sequences for comparison are well known in the art. Various programs and alignment algorithms are described in: Smith and Waterman (Adv. Appl. Math. 2: 482, 1981); Needleman and Wunsch (J. Mol. Biol. 48: 443, 1970); Pearson and Lipman (PNAS USA 85: 2444, 1988); Higgins and Sharp (Gene, 73: 237-244, 1988); Higgins and Sharp (CABIOS 5: 151-153, 1989); Corpet et al. (Nuc. Acids Res. 16: 10881-10890, 1988); Huang et al. (Comp. Appls Biosci. 8: 155-165, 1992); and Pearson et al. (Meth. Mol. Biol. 24: 307-31, 1994). Altschul et al. (Nature Genet., 6: 119-129, 1994) presents a detailed consideration of sequence alignment methods and homology calculations.

The alignment tools ALIGN (Myers and Miller, CABIOS 4:11-17, 1989) or LFASTA (Pearson and Lipman, 1988) may be used to perform sequence comparisons (Internet Program © 1996, W. R. Pearson and the University of Virginia, fasta20u63 version 2.0u63, release date December 1996). ALIGN compares entire sequences against one another, while LFASTA compares regions of local similarity. These alignment tools and their respective tutorials are available on the Internet at the NCSA Website. Alternatively, for comparisons of amino acid sequences of greater than about 30 amino acids, the Blast 2 sequences function can be employed using the default BLOSUM62 matrix set to default parameters, (gap existence cost of 11, and a per residue gap cost of 1). When aligning short peptides (fewer than around 30 amino acids), the alignment should be performed using the Blast 2 sequences function, employing the PAM30 matrix set to default parameters (open gap 9, extension gap 1 penalties). The BLAST sequence comparison system is available, for instance, from the NCBI web site; see also Altschul et al., J. Mol. Biol. 215:403-410, 1990; Gish. & States, Nature Genet. 3:266-272, 1993; Madden et al. Meth. Enzymol. 266:131-141, 1996; Altschul et al., Nucleic Acids Res. 25:3389-3402, 1997; and Zhang & Madden, Genome Res. 7:649-656, 1997.

Orthologs of proteins are typically characterized by possession of greater than 75% sequence identity counted over the full-length alignment with the amino acid sequence of specific protein using ALIGN set to default parameters. Proteins with even greater similarity to a reference sequence will show increasing percentage identities when assessed by this method, such as at least 80%, at least 85%, at least 90%, at least 92%, at least 95%, or at least 98% sequence identity. In addition, sequence identity can be compared over the full length of particular domains of the disclosed peptides.

When significantly less than the entire sequence is being compared for sequence identity, homologous sequences will typically possess at least 80% sequence identity over short windows of 10-20 amino acids, and may possess sequence identities of at least 85%, at least 90%, at least 95%, or at least 99% depending on their similarity to the reference sequence. Sequence identity over such short windows can be determined using LFASTA; methods are described at the NCSA Website. One of skill in the art will appreciate that these sequence identity ranges are provided for guidance only; it is entirely possible that strongly significant homologs could be obtained that fall outside of the ranges provided.

An alternative indication that two nucleic acid molecules are closely related is that the two molecules hybridize to each other under stringent conditions. Stringent conditions are sequence-dependent and are different under different environmental parameters. Generally, stringent conditions are selected to be about 5°C to 20°C lower than the thermal melting point I for the specific sequence at a defined ionic strength and pH. The Tₘ is the temperature (under defined ionic strength and pH) at which 50% of the target sequence hybridizes to a perfectly matched probe. Conditions for nucleic acid hybridization and calculation of stringencies can be found in Sambrook et al. (In Molecular Cloning: A Laboratory Manual, Cold Spring Harbor, New York, 1989) and Tijssen (Laboratory Techniques in Biochemistry and Molecular Biology Part I, Ch. 2, Elsevier, New York, 1993).

Hybridization conditions resulting in particular degrees of stringency will vary depending upon the nature of the hybridization method of choice and the composition and length of the hybridizing nucleic acid sequences. Generally, the temperature of hybridization and the ionic strength (especially the Na⁺ concentration) of the hybridization buffer will determine the stringency of hybridization, though waste times also influence stringency. Calculations regarding hybridization conditions required for attaining particular degrees of stringency are discussed by Sambrook et al. (ed.), Molecular Cloning: A laboratory Manual, 2nd ed., vol. 1-3, Cold Spring Harbor Laboratory Press, Cold Spring Harbor, NY, 1989, chapters 9 and 11. The following is an exemplary set of hybridization conditions:

### Very High Stringency (detects sequences that share 90% identify)

- Hybridization:: 5x SSC at 65°C for 16 hours
- Wash twice:: 2x SSC at room temperature (RT) for 15 minutes each
- Wash twice:: 0.5x SSC at 65°C for 20 minutes each

*High Stringency (detects sequences that share 80% identity or greater)*

| | |
|---|---|
| Hybridization: | 5x-6x SSC at 65°C-70°C for 16-20 hours |
| Wash twice: | 2x SSC at RT for 5-20 minutes each |
| Wash twice: | 1x SSC at 55°C-70°C for 30 minutes each |

*Low Stringency (detects sequences that share greater than 50% identity)*

| | |
|---|---|
| Hybridization: | 6x SSC at RT to 55°C for 16-20 hours |
| Wash at least twice: | 2x-3x SSC at RT to 55°C for 20-30 minutes each. |

Nucleic acid sequences that do not show a high degree of identity may nevertheless encode similar amino acid sequences, due to the degeneracy of the genetic code. It is understood that changes in nucleic acid sequence can be made using this degeneracy to produce multiple nucleic acid sequences that each encode substantially the same protein.

Specifically hybridizable and specifically complementary are terms that indicate a sufficient degree of complementarity such that stable and specific binding occurs between the oligonucleotide (or its analog) and the DNA or RNA target. The oligonucleotide or oligonucleotide analog need not be 100% complementary to its target sequence to be specifically hybridizable. An oligonucleotide or analog is specifically hybridizable when binding of the oligonucleotide or analog to the target DNA or RNA molecule interferes with the normal function of the target DNA or RNA, and there is a sufficient degree of complementarity to avoid non-specific binding of the oligonucleotide or analog to non-target sequences under conditions where specific binding is desired, for example under physiological conditions in the case of *in vivo* assays or systems. Such binding is referred to as specific hybridization.

**Subject**: Living multi-cellular vertebrate organisms, a category that includes both human and non-human mammals. This term encompasses both known and unknown individuals, such that there is no requirement that a person working with a sample from a subject know who the subject is, or even from where the sample was acquired.

**Target sequence:** "Target sequence" is a portion of ssDNA, dsDNA or RNA that, upon hybridization to a therapeutically effective oligonucleotide or oligonucleotide analog, results in the inhibition of expression. Either an antisense or a sense molecule can be used to target a portion of dsDNA, since both will interfere with the expression of that portion of the dsDNA. The antisense molecule can bind to the plus strand, and the sense molecule can bind to the minus strand. Thus, target sequences can be ssDNA, dsDNA, and RNA.

**Transformed:** A transformed cell is a cell into which has been introduced a nucleic acid molecule by molecular biology techniques. As used herein, the term transformation encompasses all techniques by which a nucleic acid molecule might be introduced into such a cell, including transfection with viral vectors, transformation with plasmid vectors, and introduction of naked DNA by electroporation, lipofection, and particle gun acceleration.

**Tumor:** A neoplasm. This term includes solid and hematological tumors.

Examples of hematological tumors include, but are not limited to: leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelogenous leukemia, and chronic lymphocytic leukemia), myelodysplastic syndrome, and myelodysplasia, polycythemia vera, lymphoma, (such as Hodgkin's disease, all forms of non-Hodgkin's lymphoma), multiple myeloma, Waldenstrom's macroglobulinemia, and heavy chain disease.

Examples of solid tumors, such as sarcomas and carcinomas, include, but are not limited to: fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, pancreatic cancer, breast cancer, lung cancer, ovarian cancer, prostate cancer, hepatocellular carcinoma, squamous cell carcinoma, basal cell carcinoma, adenocarcinoma, sweat gland carcinoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, bladder carcinoma, melanoma, and CNS tumors (such as a glioma, astrocytoma, medulloblastoma, craniopharyogioma, ependymoma, pinealoma, hemangioblastoma, acoustic neuroma, oligodendroglioma, menangioma, meningioma, neuroblastoma and retinoblastoma).

**Tumor-associated antigen or tumor antigen (TAA):** A tumor antigen which can stimulate tumor-specific T cell-defined immune responses or antibodies to tumor cells. An immunogenic composition, such as a cancer vaccine, can include one or more TAAs.

**Under conditions sufficient for/to:** A phrase that is used to describe any environment that permits the desired activity.

**Vaccine:** An immunogenic composition that can be administered to a mammal, such as a human, to confer immunity, such as active immunity, to a disease or other pathological condition. Vaccines can be used prophylactically or therapeutically. Thus, vaccines can be used reduce the likelihood of developing a disease (such as a tumor or pathological infection) or to reduce the severity of symptoms of a disease or condition, limit the progression of the disease or condition (such as a tumor or a pathological infection), or limit the recurrence of a disease or condition (such as a tumor). In particular embodiments, a vaccine is a replication-deficient CMV expressing a heterologous antigen, such as a tumor associated antigen derived from a tumor of the lung, prostate, ovary, breast, colon, cervix, liver, kidney, bone, or a melanoma.

**Vector:** Nucleic acid molecules of particular sequence can be incorporated into a vector that is then introduced into a host cell, thereby producing a transformed host cell. A vector may include nucleic acid sequences that permit it to replicate in a host cell, such as an origin of replication. A vector may also include one or more selectable marker genes and other genetic elements known in the art, including promoter elements that direct nucleic acid expression. Vectors can be viral vectors, such as CMV vectors. Viral vectors may be constructed from wild type or attenuated virus, including replication deficient virus.

In particular non-limiting examples, the viral vector is a replication deficient CMV lacking a necessary glycoprotein such as gB, gD, gH, or gL.

Vectors can also be non-viral vectors, including any plasmid known to the art.

**Virus**: Microscopic infectious organism that reproduces inside living cells. A virus consists essentially of a core of a single nucleic acid surrounded by a protein coat (capsid), and has the ability to replicate only inside a living cell. "Viral replication" is the production of additional virus particles by the occurrence of at least one viral life cycle. A virus may subvert the host cells' normal functions, causing the cell to behave in a manner determined by the virus. For example, a viral infection may result in a cell producing a cytokine, or responding to a cytokine, when the uninfected cell does not normally do so.

In a "lytic" or "acute" viral infection, the viral genome is replicated and expressed, producing the polypeptides necessary for production of the viral capsid. Mature viral particles exit the host cell, resulting in cell lysis. Particular viral species can alternatively enter into a "'lysogenic" or "latent" infection. In the establishment of latency, the viral genome is replicated, but capsid proteins are not produced and assembled into viral particles.

Unless otherwise explained, all technical and scientific terms used herein have the same meaning as commonly understood by one of ordinary skill in the art to which this invention belongs. The singular terms "a," "an," and "the" include plural referents unless context clearly indicates otherwise. Similarly, the word "or" is intended to include "and" unless the context clearly indicates otherwise. Hence "comprising A or B" means including A, or B, or A and B. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of the present invention, suitable methods and materials are described below.

### III. Overview of Several Embodiments

Described herein are methods of generating a long term, repeatedly stimulated immune response against a heterologous antigen in a subject comprising: administering to the subject, e.g. by intraperitoneal or intravenous administration (such as a single administration), a recombinant, replication-deficient cytomegalovirus comprising a heterologous nucleic acid encoding the antigen, whereby viral latency is established in the subject, which latency results in the repeatedly stimulated immune response against the antigen. In particular embodiments, the repeatedly stimulated immune response comprises a CD8⁺ T cell immune response. The heterologous antigen is a cancer antigen.

In yet further examples, the recombinant replication-deficient cytomegalovirus comprises an inactivated gB, gD, gH, or gL glycoprotein gene, such as a gL glycoprotein that is inactivated by a knock out mutation.

In particular embodiments, the nucleic acid encoding the antigen is operably linked to a constitutive promoter. In other embodiments, the nucleic acid encoding the antigen is operably linked to an inducible promoter.

In some embodiments of the described methods, the recombinant replication-deficient cytomegalovirus is a murine cytomegalovirus. In other embodiments, the recombinant replication-deficient cytomegalovirus is a human cytomegalovirus, such as a AD169, Davis, Toledo or Towne strain of CMV.

Also described herein are methods of treating a subject who has been diagnosed with a cancer comprising: administering to the subject one or a combination of a chemotherapeutic or a immunologic anti-cancer agent; and administering to the subject, by intraperitoneal or intravenous administration, a recombinant, replication-deficient cytomegalovirus comprising a heterologous nucleic acid encoding a heterologous antigen derived from the cancer, whereby viral latency is established in the subject, thereby generating repeatedly stimulated immunity against the cancer.

In particular of these methods, the repeatedly stimulated immunity comprises a CD8⁺ T cell immune response. In other methods, the recombinant replication-deficient cytomegalovirus comprises an inactivated gB, gD, gH, and gL glycoprotein gene, such as a gL glycoprotein that is inactivated by a knock out mutation.

In some embodiments, the recombinant replication-deficient cytomegalovirus is a human cytomegalovirus, such as a AD169, Davis, Toledo, or Towne strain of CMV.

In yet further examples, the described methods of treating a subject who has been diagnosed with a cancer further comprise administering radiation therapy to the subject.

### IV. Generation of long-term repeatedly stimulated immune response with replication-deficient CMV

Infection by CMV proceeds in two distinct phases that are discernable by measurable changes in virus titer and host immune response (Figure 1). Similar to infections by other virus species, initial CMV titer sharply increases in an acute phase of infection, indicating active viral reproduction and cell lysis. Likewise, host immune response, as illustrated by percentage of CMV-reactive T cells, sharply increases. While this CMV-triggered immune response results in the clearance of the acute infection, CMV is not eradicated from the host. Instead, the virus establishes latency in particular cells types, including endothelial cells and CD34⁺ hematopoietic stem cells (HSCs) (Sissons et al., Journal of Infection 44:73-77, 2002). Viral titer is usually below the limit of detection during latent infection. In healthy individuals, occasional recurrence of acute CMV infection is rapidly cleared by the remarkably large percentage of memory T cells that are CMV-reactive. Indeed, it has been shown that following the initial acute infection, the CD8⁺ T cell response to CMV in both mouse and humans undergoes "memory inflation", occupying about 10% of the CD8⁺ T cell memory compartment for the life of the host *(*Karrer et al., J. Immunol. 170:2022-2029, 2003; Sylwester et al, J. Exp. Med. 202:673-685, 2005; Gillespie et al., J. Virol. 74:8140-8150, 2000). Most of these "inflated" CMV-specific memory T cells have a tissue-patrolling effector memory phenotype and immediate effector capability (Bitmansour et al., J. Immunol. 169:1207-1218, 2002). The large numbers and immediate effector capabilities of these cells indicate that they are being repeatedly stimulated. This is attributed to the fact that CMV repeatedly undergoes small, rapidly controlled recurrences of active virus infection (Wherry and Ahmed, J. Virol. 78:5535-5545,2004). Thus, the current view regards the repeatedly stimulated phenotype as inextricably linked to replication competent virus.

Disclosed herein is the surprising observation that a replication-deficient CMV that is completely unable to generate infectious viral particles, and so cannot spread between cells, is nevertheless capable of eliciting CD8⁺ T cell memory inflation, indicative of repeated stimulation, when administered to a subject by a single intraperitoneal or intravenous dose. T cells elicited by this method have the tissue tracking, effector-memory phenotype and can provide immediate protection against antigen in the tissues.

The inherent danger to immunocompromised individuals of administering a replication-competent CMV-vectored vaccine is well known. One of skill in the art will therefore recognize the benefits of the herein-described extraordinary finding that a completely replication-incapable CMV can produce a CMV-reactive CD8⁺ T cell immune response, and how this discovery can be used to develop universally safe CMV-vectored immunogenic compositions.

Thus, provided herein are recombinant replication-deficient CMV virus vectors that encode a heterolocrous cancer antigen that serves as an immune stimulant (for instance, a vaccine), to elicit a long-term repeatedly stimulated immune response to the heterologous antigen. These engineered immune stimulating viruses can be used to elicit a long-term, repeatedly stimulated immune response against tumors.

Described herein are methods of generating a long-term repeatedly stimulated immune response against a heterologous antigen in a subject. The methods involve one-time e.g. intraperitoneal or intravenous administration of a recombinant replication-deficient CMV that includes a heterologous polynucleotide encoding the heterologous antigen. This one time administration is sufficient to repeatedly stimulate the immune system to elicit a long-term, activated CD8⁺ T cell based immunity against the antigen, and by extension, pathogens or tumors expressing the antigen.

### Replication-deficient CMV

The methods described herein employ a CMV that is completely unable to spread beyond the initial host cell. Thus, contemplated herein is the use of any replication-incompetent CMV that can establish latency and thereby provide repeated stimulation to the immune system (even with a single administration of the vector), but that cannot produce infectious viral particles. In particular non-limiting examples, the CMV is replication-deficient because it comprises an inactivated (e.g., knocked- out) gB, gD, gH, or gL glycoprotein gene. In other examples, the CMV comprises multiple inactivated essential genes (which may or may not include one or more glycoprotein gene) required for generation of infectious viral particles.

Methods of producing an inactivating gene mutation (including, but not limited to "knock-out" mutations) are well known to the art (Ausubel et al. (eds.), Current Protocols in Molecular Biology, published by Wiley InterScience, 2009 (ISSN 1934-3639)). One such method for mutating a nucleic acid is to clone the nucleic acid, or a part thereof, modify the sequence of the nucleic acid by site directed or random mutagenesis, and reintroduce the mutated nucleic acid into the viral genome, for instance by homologous recombination.

Another method for inactivating an essential CMV replication gene is through deleting all or part of the genome-resident copy of the gene. In one example, the entire gene is deleted (*i.e.,* a knockout mutation), the deletion occurring in such as way as to make reversion to a functional form of the gene essentially impossible. In another embodiment, a partial deletion is produced. In another embodiment, the portion of the nucleic acid encoding amino acid residues necessary for polypeptide function is deleted (for example, those gL amino acids necessary for its interaction with gH).

In particular examples, the essential CMV replication protein, such as the gB, gD, gH, or gL glycoprotein, is inactivated by knockout deletion of the gene. Knockout deletion of a target gene, using the gL glycoprotein as an illustrative example, can be carried out as follows: The CMV gL gene sequence is isolated *(e.g.,* by amplification of viral DNA) and inserted into a cloning vector, including a portion of nucleic acid sequence upstream and downstream of the coding sequence of the gL gene itself. The coding region of the gene is then deleted from the vector *in vitro,* leaving behind a sufficient length of the 5' and 3' flanking sequence to permit homologous recombination with the naturally occurring gene in the viral genome. The modified vector is then transformed into a CMV-infected cell where the knockout mutation integrates into the CMV genome via homologous recombination in the flanking regions. Another method uses the modified vector to transform bacterial cells expressing the CMV genome in a bacterial artificial chromosome (BAC) (Borst et al., in Current Protocols in Immunology, Ch. 10, Unit 10.32, published by Wiley InterScience, 2009 (ISSN 1934-3671). The recombinant BAC is then transfected into permissive cells to generate the recombinant virus. These methods result in a viral strain in which the gL gene has been deleted, and which can only be propagated by complementation of the defective gL - for instance, by infection of host cells that simultaneously express the gL protein, or in the presence of helper virus that expresses the gL protein. In particular embodiments, the nucleic acid sequence encoding the target gene for inactivation is substituted with a marker such as lacZ, luciferase, or green fluorescent protein. Such markers are not intended to provoke a host immune response, but instead can be utilized to identify recombinant viruses.

Another method of inactivating a naturally occurring gene is to mutagenize the genomic copy of the gene *in situ,* for instance, by transforming an infected cell with mutagenic oligonucleotides *(see* for example the method of Storici et al., Nature Biotech. 19:773-776, 2001).

Any replication-deficient CMV is contemplated for use in the methods described herein; likewise, different strains of CMV can be used for generating a replication-deficient CMV vector as described herein. In particular embodiments, the CMV is a murine CMV (mCMV). In other embodiments it is a non-human primate CMV, such as a rhesus macaque CMV. In other embodiments, it is a human CMV (hCMV). In still other examples, the replication-deficient CMV is based on the AD169, Davis, Toledo, and Towne strains of CMV, or any human CMV strain derived therefrom.

### Heterologous Antigens

The methods described herein involve use of a replication deficient CMV vector as the basis for an immunogenic composition to generate a long term, repeatedly stimulated immune response in a subject against a heterologous cancer antigen that is expressed by the CMV vector. Generation of this long-term immune response simultaneously produces immediate protection against a tumor (by virtue of a CTL killing effect) as well as the life-long immunity described herein.

The heterologous antigen used in the described methods is an antigenic polypeptide encoded by a heterologous polynucleotide that is incorporated into the genome of the replication-deficient CMV. In particular examples, the antigenic polypeptide is derived from a tumor cell.

The antigenic polypeptide sequence may be any length sufficient to elicit the immune response. In particular examples, the polypeptide is at least 10, at least 20, at least 30, at least 40, at least 50 amino acids long or greater. Likewise, the sequence identity of the antigenic polypeptide need not be identical to the sequence identity to the native polypeptide in order to be sufficient to maintain specificity of the immune response against the tumor. One of skill in art will recognize that the sequence of a polypeptide may be significantly altered while maintaining its antigenic specificity - that is, the ability to stimulate an antigenic response that will still provide responsiveness to the native protein. Thus, in particular examples, the antigenic polypeptide is at least 50%, at least 60%, at least 70%, at least 80%, at least 90%, at least 95%, or at least 98% identical to the polypeptide from which it was derived.

The heterologous polynucleotide carried by the replication-deficient CMV encodes a polypeptide derived from a tumor. The tumor may be the result of any type of cellular proliferative disease or condition, and may be benign or malignant. In particular examples, the tumor is cancerous. Such tumors can be of any type of cancer including, but not limited to: leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelogenous leukemia, and chronic lymphocytic leukemia), myelodysplastic syndrome, and myelodysplasia, polycythemia vera, lymphoma, (such as Hodgkin's disease, all forms of non-Hodgkin's lymphoma), multiple myeloma, Waldenstrom's macroglobulinemia, and heavy chain disease. Examples of solid tumors, such as sarcomas and carcinomas, include, but are not limited to: fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, pancreatic cancer, breast cancer, lung cancer, ovarian cancer, prostate cancer, hepatocellular carcinoma, squamous cell carcinoma, basal cell carcinoma, adenocarcinoma, sweat gland carcinoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, bladder carcinoma, melanoma, and CNS tumors (such as a glioma, astrocytoma, medulloblastoma, craniopharyogioma, ependymoma, pinealoma, hemangioblastoma, acoustic neuroma, oligodendroglioma, menangioma, meningioma, neuroblastoma and retinoblastoma).

Antigenic tumor-derived polypeptides that may be encoded by the heterologous polynucleotide of the described methods encompass polypeptides also known as tumor associated antigens (TAAs), and peptides derived therefrom. Many TAAs have been identified. These include, but are not limited to: human telomerase reverse transcriptase (hTERT), survivin, MAGE-1, MAGE-3, human chorionic gonadotropin, carcinoembryonic antigen, alpha fetoprotein, pancreatic oncofetal antigen, MUC-1, CA 125, CA 15-3, CA 19-9, CA 549, CA 195, prostate-specific antigens; prostate-specific membrane antigen, Her2/neu, gp-100, trp-2, mutant K-ras proteins, mutant p53, truncated epidernal growth factor receptor, chimeric protein .sup.p210BCR-ABL; E7 protein of human papilloma virus, and EBNA3 protein of Epstein-Barr virus (these TAAs and others are described in Novellino et al., Cancer Immunology and Immunotherapy, 54:187-207, 2005).

One of ordinary skill will recognize that the lists of exemplary heterologous polypeptides discussed herein are neither exhaustive nor intended to be limiting. Thus, it will be recognized that the methods provided herein are useful for expression of, and thus immune-stimulation related to, any polypeptide against which it would be beneficial to generate long-term self priming immunity.

### CMV latency and expression of heterologous antigen

In addition to other cell types, CMV establishes latency in CD34⁺ hematopoietic stem cells (HSCs) (Sinclair, J. Clin. Virol. 41:180-185, 2008 and Mocarski et al., in Cytomegaloviruses: Moleoular Biology and Immunology. M. J. Reddehase, ed., published by Caister Academic Press, Wymondham, U.K., pp. 464-482, 2006). Although CMV does not contain any known latency-specific genes, the CMV genome is maintained for life in HSCs. The surprising observation provided herein that a replication-deficient CMV can elicit a long-term, repeatedly stimulated immune response suggests that as progeny of HSCs divide, the CMV genome hosted therein is also replicated and passed to daughter cells *without the production of infectious virus particles.* While not intending to be bound by a single theory, it is proposed that HSC-hosted (or other-cell-hosted) latency is a likely explanation for the ability of the replication-deficient CMV to persist and stimulate memory inflation. As HSCs differentiate into mature monocytes and dendritic cells, CMV reactivates and expresses its full complement of genes in a regulated cascade of three gene classes: immediate early (IE), early (E), Late (L) (Mocarski and Courcelle, in Fields Virology, Vol. 2. D. M. Knipe, and P. M. Howley, eds., published by Lippincott Williams and Wilkins, Philadelphia, p. 2629-2674, 2001). However, latently infected cells can also undergo an abortive replication cycle in which only EE genes are expressed Reddehase et al., Journal of Clinical Virology 25 Suppl 2:523-36, 2002; Kurz et al., Journal of Virology 71:2980-2987, 1997; Kurz et al., Journal of Virology 73:8612-8622, 1999). Abortive reactivation is far more common than full replicative cycles. Thus, the immune system is repeatedly exposed to IE antigens in the context of the most potent of all antigen-presenting cells.

In the immune-stimulation methods described herein, the heterologous polynucleotide that encodes the antigen is inserted into a replication-deficient CMV genome (a CMV vector). Methods of generating recombinant viral vectors are well known in the art (*see* Ausubel et al. (eds.), Current Protocols in Molecular Biology, published by Wiley InterScience, 2009 (ISSN 1934-3639), and (Borst et al., in Current Protocols in Immunology, Ch. 10, Unit 10.32, published by Wiley InterScience, 2009 (ISSN 1934-3671). One such method for inserting a heterologous polynucleotide into a viral genome is to clone the polynucleotide of interest between a sufficient length of 5' and 3' viral genomic flanking sequence to permit homologous recombination with the naturally occurring viral genomic sequence. The resultant targeting vector is then transformed into a bacterial cell expressing the CMV genome as a BAC, where the polynucleotide integrates into the virus genome via homologous recombination in the flanking regions.

The heterologous polynucleotide may be operably linked to any promoter that will drive expression of the heterologous antigen to elicit an immune response. The polynucleotide may be operably linked to such promoters either by way of insertion of the polynucleotide into the viral genome or in the process of cloning the polynucleotide. In particular examples, the promoter may be a CMV promoter such as the CMV major immediate early promoter (MIEP). Epitopes expressed behind murine IE promoters elicit immunodominant, inflating T cell responses in mice (Karrer et al., J. Immunol. 170:2022-2029, 2003; Munks et al., J. of Immunol. 177:450-458, 2006; Holtappels et al., J. Virol. 74:11495-11503, 2000; and Karrer et al., J. Virol. 78:2255-2264,2004). Similarly in humans, IE-encoded epitopes are favored disproportionately during latent infection (Sylwester et al., J. Exp. Med. 202:673-68, 2005).

In other embodiments, the heterologous polynucleotide is operably linked to a constitutive promoter that continually recruits the cellular transcriptional machinery and initiates transcription (Fitzsimons et al., Methods. 28:227-236, 2002). In a particular example, the heterologous polynucleotide is operably lined to a strong constitutive promoter such as the human elongation factor 1 alpha (EF1-a) promoter (*see* Kim et al., Exp. Mol. Med., 37:36-44, 2005). In other examples, the heterologous polynucleotide is operably linked to an inducible promoter that requires particular conditions or additional factors for initiation of transcription (Guo et al., Trends Mol Med. 14:410-418, 2008).

Detailed methods of constructing recombinant viral vectors are described in U.S. Patent Publication 2005/0118192 and U.S. Patent Publication 2008/0044384.

### V. Immunogenic preparations

In particular embodiments of the described methods, the recombinant replication deficient CMV comprising a heterologous polynucleotide encoding an antigen is an immunogenic preparation that is administered to a subject. The immunogenic preparations will generally comprise one or more pharmaceutical compositions. Immunogenic preparations are generally described in, for example, M.F. Powell and M.J. Newman, eds., "Vaccine Design (the subunit and adjuvant approach)," Plenum Press (NY, 1995).

Though not necessary to generate the long term, repeatedly stimulated immune response described herein, the immunogenic preparations may optionally comprise an immunostimulant such as an adjuvant. Adjuvants could include, but are not limited to, CpG oligonucleotides, or cytokines, such as GM-CSF or interleukin-12, or antibodies such as anti-CTLA4.

In particular examples, the immunogenic preparations may contain pharmaceutically acceptable carriers. While any suitable carrier known to those of ordinary skill in the art may be employed in the immunogenic preparations of this invention, the type of carrier will vary depending on the mode of administration. Immunogenic preparations for practicing the described methods are formulated for intravenous or intraperitoneal administration. Under ordinary conditions of storage and use, these preparations may contain a preservative to prevent the growth of microorganisms.

The pharmaceutical forms of the immunogenic preparations suitable for injectable use include sterile aqueous solutions or dispersions. In all cases the form must be sterile and must be fluid to the extent that it will pass easily through a syringe. The immunogenic preparation must also be stable under the conditions of manufacture and storage and must be preserved against the contaminating action of microorganisms, such as bacteria and fungi.

Such preparations may also comprise buffers (*e.g.*, neutral buffered saline or phosphate buffered saline), carbohydrates (*e.g.*, glucose, mannose, sucrose or dextrans), mannitol, proteins, polypeptides or amino acids such as glycine, antioxidants, bacteriostats, solutes that render the formulation isotonic, hypotonic or weakly hypertonic with the blood of a recipient, suspending agents, thickening agents and/or preservatives. Alternatively, the immunogenic preparations for use in the present invention may be formulated as a lyophilizate.

Some variation in dosage will necessarily occur depending on the condition of the subject being treated. The person responsible for administration will, in any event, determine the appropriate dose for the individual subject. A suitable dose is an amount of the immunogenic preparation that, when administered as described above, is capable of promoting an immune response against the viral vectored heterologous antigen above the basal (*i.e*., untreated) level. Suitable doses in certain embodiments range from one thousand to 10 million plaque forming units.

The response to the immunogenic preparation can be monitored, for example, by measuring the percent of heterologous antigen-reactive T cells present in a patient or the stimulation of γIFN production in response to the antigen. With generation of long-term, repeatedly stimulated immunity, a response to antigen stimulation will be measurable weeks and years after administration of the immunogenic preparation.

Use of an immunogenic preparation for practicing the methods described here should also be capable of causing an immune response that leads to an improved clinical outcome (*e*.*g*., more frequent remissions, complete or partial or longer disease-free survival) in vaccinated patients as compared to non-vaccinated patients.

In particular example, establishment of latency can be determined by detection or genomic DNA in a host (*e*.*g*., by PCR amplification of viral DNA) in the absence of detectable virus (*see* Liu et al., J. Virol 82:10922-10931, 2008). Other methods of detecting or determining latency will be known to those of skill in the art.

### VI. Combination methods for cancer treatment

The ability to generate a long-term, repeatedly stimulated immune response with a CMV vector that is completely incapable of spreading between cells is beneficial in the context of cancer therapy. Both radiation and chemotherapy treatments are directed to killing rapidly dividing cells. However, many micrometastases are not rapidly dividing and so survive these treatments. Similarly, many immunologic, for example antibody-based, treatments are short-lived and may not completely eradicate all of the cancer cells in a patient.

A particular embodiment of the methods described herein addresses the deficiencies of traditional cancer treatment. This embodiment involves administering to a subject one or a combination of a chemotherapeutic or a immunologic anti-cancer agent or radiation therapy; and administering to the subject by intraperitoneal or intravenous administration, a recombinant, replication-deficient cytomegalovirus comprising a heterologous nucleic acid encoding a heterologous antigen derived from the cancer, whereby viral latency is established in the subject, thereby repeatedly stimulating immune cells to generate long-term immunity against the cancer.

Any form of cancer, such as those described herein, can be treated using the described combination methods. Likewise, a polynucleotide encoding any cancer antigen, such as those described herein, may be used to generate the long-term, repeatedly stimulated immune response of the described methods.

Additionally, methods of radiation therapy, immunologic treatment and chemotherapy are all well known to the art *(see* Abeloff, *Clinical Oncology* 2^{nd} ed., 2000 Churchill Livingstone, Inc). For example, a subject diagnosed with head and neck squamous cell carcinoma may be administered a chemotherapeutic agent, such as cisplatin (Cooper et al., N. Engl. J. Med. 350(19):1937-1944, 2004). Other types of cancer also respond to cisplatin therapy, such as testicular cancer, ovarian cancer or other reproductive cancers.

In another example, a subject diagnosed with multiple myeloma is administered a chemotherapeutic (ICL-inducing) agent, such as melphalan. A similar treatment regimen can be used for patients with other types of cancer that respond to melphalan therapy, such as ovarian cancer and colorectal cancer.

In another example, a subject diagnosed with a lymphoma is administered an ICL-inducing agent, such as Cytoxan^{™}. The lymphoma can be any type of lymphoma that responds to treatment with Cytoxan^{™}, such as Hodgkin's lymphoma or non-Hodgkin's lymphoma. A similar treatment regimen can be used for patients with other types of cancer that respond to Cytoxan^{™} therapy, such as breast cancer, multiple myeloma, retinoblastoma, ovarian cancer, neuroblastoma and leukemia (including acute lymphoblastic leukemia, acute myeloid leukemia, chronic lymphocytic leukemia and chronic myelogenous leukemia).

In another example, a subject in need of bone marrow transplantation/conditioning for the treatment of chronic myelogenous leukemia or chronic lymphocytic leukemia is administered busulfan.

The cancers that are chemotherapeutically-treated in the above examples may alternatively or additionally be treated by any other means known to the art of eradicating cancer from a subject. Such methods include surgical removal of cancerous tissue, radiation therapy, or immunotherapy.

The following examples are provided to illustrate certain particular features and/or embodiments. These examples should not be construed to limit the invention to the particular features or embodiments described.

### EXAMPLES

### Example 1: Anti-tumor efficacy of CTL elicited by a CMV-vectored antigen

A major challenge in developing an effective anti-tumor vaccine is overcoming the inability of the immune system to distinguish between tumor and self proteins. Recently, efforts to develop a transmissible immunocontraceptive for control of feral mice demonstrated that that a normal self protein expressed in a recombinant CMV vector could become antigenic (Lloyd et al., Biol. Reprod. 68:2024-2032, 2003 and Redwood et al., J. Virol. 79:2998-3008, 2005). In that study, the native sequence for murine zona pellucida 3 (ZP3), a normal mouse protein, was introduced into MCMV behind the immediate early 2 (IE2) promoter. Strikingly, a single inoculation of this virus renders female mice completely infertile for as long as they have been observed (>230 days).

This example demonstrates that tumor-expressed self antigens also become immunogenic when expressed in the context of CMV. The therapeutic ability of the CMV-vector elicited immune response is also demonstrated and shows the potential efficacy of a CMV-vector anti-tumor immunogenic preparation. In this example, MCMV expressing ovalbumin (ova) was used to immunize mice, and the CD8⁺ T cell response to ovalbumin was measured six months later. They were then challenged with an aggressive tumor that had been transfected with ovalbumin.

### Methods

*Infection of Mice.* C57BL/6 mice were injected intraperitoneally with MCMV-IE2-OVA (Snyder et al., Immunity, 29:650-659, 2009), or with control wildtype MCMV at 10⁶ pfu per mouse. Additional control mice were uninfected. Six months later, mice were bled for analysis of the T cell response to OVA, and subjected to tumor challenge. *Intracellular cytokine staining.* To prepare the cells for stimulation, mice were bled from the lateral tail vein into a collection tube containing 12 µl of heparin to prevent clotting. Blood was transferred into 14 ml tubes and 3 ml of red cell lysis buffer (150mM NH₄Cl, 10mM NaHCO₃) were added to each sample. Samples were mixed and incubated for 3 minutes at room temperature. Following red cell lysis, 9 mls of T cell media (RPMI, 10% Fetal Calf Serum, 100 Units/ml penicillin, 100 µg/ml streptomycin, 20 mM glutamine, 5x10⁻⁵ M β-mercaptoethanol) were added to each tube and cells were spun at 500 x g for 10 minutes. The supernatant was removed by vacuum suction and the cell pellet was resuspended in 250 µl of fresh T cell media. Finally, 50 µl of T cells were aliquoted into replicate wells of a 96-well, round-bottom plate. To prepare the peptides for stimulation, the indicated peptides were diluted to 2 µg/ml (~2 µM) in fresh T cell media (prior to dilution, peptide stock solutions were maintained at 2 mg/ml in 100% DMSO at -20°C.). For unstimulated wells, the same volume of DMSO was added to T cell media in place of peptide. Brefeldin A (We used Golgi-Plug from CD Biosciences) was then diluted to a concentration 2 µg/ml (1:500 from the stock tube of Golgi-Plug) into the each peptide mixture and the entire solution was mixed carefully. To stimulation the T cells, 50µl of the peptide/Golgi-Plug mixture was added to the 50µl of peripheral blood cells already in the 96-well plate. Samples were mixed gently and incubated in a humidified incubator at 37°C + 5% CO₂ for 6 hours. After the 6hr incubation, cells were chilled on ice for 5 minutes. Subsequently, 100µl of ice cold FACS buffer (PBS; 1% Fetal Calf Serum; 0.01% Sodium Azide) was added to each well and the plates were spun cells at 500 x g for 3 minutes at 4°C. Following the spin, supernatant was dumped off and cells were resuspended on ice with a mixture of ice cold FACS buffer and anti-CD8 antibody (clone 53.6-7). Cells and antibody were incubated overnight at 4°C. The following morning, the cells were washed 3 times. Each wash consisted of adding ice cold FACS buffer and spinning the cells at 500 x g for 3 minutes at 4°C. To keep the cells cold, they were maintained on ice between each spin. Following the 3rd wash, cells were fixed at 4°C for 10 minutes in 80 µl of Fix/Perm solution from BD Biosciences. Subsequently, cells were washed twice (as above) with 1x Perm/Wash buffer from BD Biosciences to remove any excess fixative. Finally, anti-IFNγ (clone XMG1.2) diluted in 1x Perm/Wash buffer was added to each sample and incubated for 1 hour at 4°C. Following the IFN-γ stain, cells were washed 3 times with 1x Perm/Wash buffer, resuspended in FACS buffer, and assayed on a BD FACScalibur flow cytometer.

*Tumor challenge.* The aggressive D5 subclone of the B16 melanoma cell line was transfected with a retroviral vector expressing ovalbumin and GFP. 48 hours later, cells were sorted by FACS for GFP bright cells. This population of cells was then counted and 20,000 cells injected subcutaneously into the flank of each of three groups of animals, 6 animals per group (a) unmanipulated control animals (b) mice infected with MCMV vector only (c) mice infected with MCMV ova. The diameter of palpable tumor was measured daily using calipers until the end of the experiment, or until mice became moribund, in which case they were euthanized.

### Results

The ability of CMV-vectored immunity to exert anti-tumor effector function, was tested using a recombinant live MCMV strain that expresses ovalbumin behind the CMV IE2 promoter (MCMV-ova (Snyder et al., Immunity, 29:650-659, 2009)). This strain of recombinant MCMV was then tested as an immunogenic preparation against a highly aggressive ova-expressing tumor.

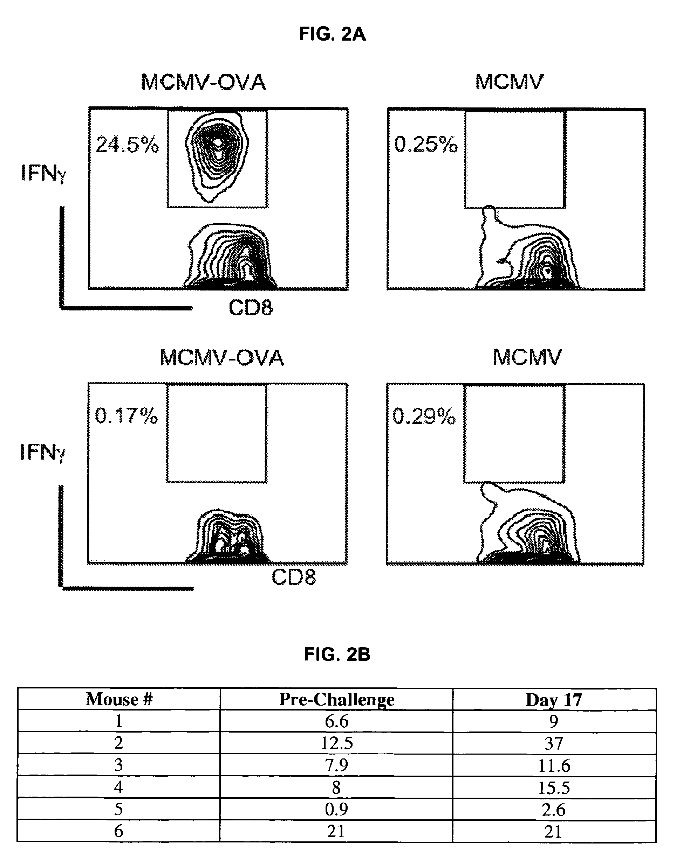

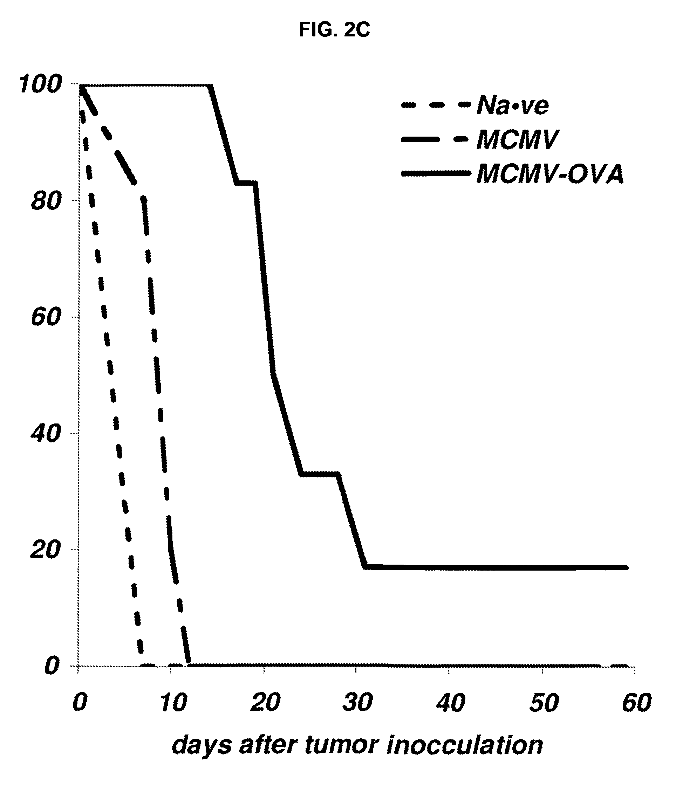

To assess the CD8⁺ T cell response to MCMV-vectored tumor antigen, MCMV-ova was administered to mice by a single intraperitoneal injection. After six months, T cell responses to MCMV-vectored antigen were detected directly *ex vivo* in the peripheral blood in normal C57BL6 mice, without use of transgenic T cells (Figure 2A and B). Six months after vaccination to MCMV-ova, the ova-specific immune response ranged from 1% to 24% of CD8⁺ T cells, and in 5 of 6 mice was >7%.

MCMV-ova-vaccinated mice were then challenged with an aggressive subclone of B 16 melanoma, which was transfected with a retroviral vector expressing ovalbumin to generate D5-OVA. D5-OVA was not cloned, and the challenge line may have contained some ova-negative cells. The tumor grew rapidly in both naive and vector-vaccinated mice (Figure 2C), with similar growth kinetics to the parental D5 clone. In contrast, in the MCMV-ova-immunized mice, the ova-specific immune response measured in the peripheral blood lymphocytes expanded, and the growth of tumor was markedly delayed. One mouse never developed tumor. Moreover, tumor cells that were obtained from two of the vaccinated mice were both antigen escape variants, and were unable to stimulate ova-specific B3Z hybridoma cells. Thus, the CMV-vectored immune response provided a powerful selective pressure for immune escape of this highly aggressive tumor, and in one case provided complete protection.

### Example 2: Infection by replication-deficient ΔgL MCMV elicits persistent immunogenic priming and T cell memory inflation

On average, humans devote 10% of their memory CD8⁺ T cell compartment to CMV. Studies in recent years have revealed this extraordinary immune response to CMV infection comprises several phases. After an initial CD8⁺ T cell response that looks similar to that elicited by acute viruses such as vaccinia, the T cell response to MCMV initially declines to characteristic "memory" levels (Munks et al., J. Immunol. 176:3760-3766, 2006. However, it then begins the remarkable process known as "memory inflation" (Karrer et al., J. Immunol. 170:2022-2029, 2003 and Munks et al., J. Immunol. 177:450-458, 2006). For a period of some months, the CD8 T cell responses to certain epitopes increase in numbers, eventually plateauing at a high level such that the total CMV-specific response comprises between 10 and 30% of CD8⁺ T cells.

This example demonstrates that a replication-defective MCMV from which the gL glycoprotein has been deleted can effectively drive the same immune response elicited by a replication competent CMV. Replication deficient CMV was used initially as a negative control for investigating the requirements for memory inflation, with the expectation that it would express no antigen and that there would therefore be no memory inflation. The results shown demonstrate the surprising finding of the opposite result: that the replication deficient virus did indeed continue to stimulate the inflationary T cell response.

### Methods

Unless otherwise specified, methods were as described in Example 1. Mice were infected with a single intraperitoneal injection of 1x10⁵ plaque forming units of wild-type MCMV or replication-deficient, ΔgL MCMV. Antigen-specific T cells were identified by both MHC-tetramer staining (Figures 3, 4 5A and 5C) and intracellular cytokine staining (Figure 5B). The intracellular cytokine staining for IFN-γ production by antigen-specific T cells was performed as described above in Example 1. The protocol for is described below.

*Tetramer staining*. To prepare the cells, mice were bled from the lateral tail vein into a collection tube containing 12 µl of heparin to prevent clotting. From each mouse, 50µl of whole blood was added into individual wells of a round bottom plate for each of the 3 tetramers shown. To prepare the tetramers for staining, each tetramer was synthesized and loaded with the indicated peptide by the NIH core facility *(see* online at niaid.nih.gov/reposit/tetramer/overview.html) and conjugated to APC (allophycocyanin) or PE (Phycoerythrin). The undiluted stock tetramer was spun for 5 minutes at 16,100 x g to remove any aggregates. To stain the T cells, each batch of tetramers had been previously titrated to identify the optimal staining concentration, which is considered a 1x concentration. After centrifugation, each tetramer was then diluted to a 3.5x concentration in FACS buffer (see example 1 for formulation) and 20 µl of the mixture was added to each blood sample in the 96-well plates. The final volume at this point is 70 µl and the final concentration of the MHC-tetramer staining reagent is 1x. Cells were incubated with the tetramers for 20 minutes at room temperature in the dark and then washed 3 times in 150µl of FACS buffer as described in example 1 above. After each spin, the supernatant was carefully drawn off of the cells with a pipette to avoid disturbing the blood pellet. Following the third wash, T cells were counterstained with antibodies to surface markers CD8 (clone 53.6-7), CD127 (clone A7R34) and KLRG-1 (clone 2F1). The antibodies were diluted into FACS buffer and mixed with blood samples before incubating for 20 minutes at room temperature in the dark. Following the surface stain, the cells were washed once with 150µl of FACS buffer and supernatant was carefully removed as above. Next, red blood cells were lysed by resuspending the cells in 200µl of 1x Red Cell Lysis Buffer from BD Biosciences, incubating for five minutes at room temperature and spinning at 500 x g. After this spin the supernatant was dumped as above in Example 1 and the cells were washed twice more in FACS buffer. In most cases, the samples were fixed in 80µl of Fix/Perm buffer from BD Biosciences to preserve cell morphology before analysis on an LSR II flow cytometer.

### Results

### The CD8 T cell response to wildtype MCMV in C57BL/6 mice

The immune response to MCMV infection was determined in C57BL/6 mice infected by intraperitoneal injection of wildtype MCMV. Figure 3 shows the CD8⁺ T cell response to CMV epitope stimulus by viral proteins seven days (Figure 3A) and twelve weeks (Figure 3B) post-infection. The development of the response to m139, M38, and IE3 shown in Figure 3B is the result of memory "inflation."

T cell memory "inflation" was tracked by tetramer staining to determine percentages of m139-, M38-, and IE3-responsive cells. Figure 4 shows the increase and plateauing of responsive cells in peripheral blood.

### CD8⁺ T cell memory inflation in response to a replication-deficient MCMV infection

The ability for a replication-defective CMV to elicit CD8⁺ T cell inflation was tested in using virus that lacks gene encoding essential envelope glycoprotein gL, a gift from Dr Jane Allen. In the ΔgL virus, the gL gene has been replaced by lacZ; and the virus is propagated by growth on a complementing cell line (3T3 cells transfected with gL). Although this virus is able to replicate its genome, it is completely unable to spread between cells.

The CD8⁺ T cell response to infection by the ΔgL virus was measured using tetramer staining of antigen-responsive cells up to 29 weeks post-infection. Surprisingly, as shown in Figure 5, the CD8⁺ T cell response to this virus displayed characteristic memory inflation, including the *de-novo* later appearance of responses to the IE3 epitope. The responses were lower than seen with wildtype virus, but still quite large. By 20 weeks post infection, the IE3-specific responses had accumulated to 1-2% of *all* CD8⁺ T cells in the peripheral blood. M38-specific and m139-specific CD8⁺ T cell responses were maintained at similar levels (Figure 5A).

The cells that accumulated after ΔgL infection secreted the effector cytokine IFN-γ (Figure 5B) after peptide stimulation, indicating that they had good, immediate effector function. For this experiment, T cells were analyzed 25 weeks post infection. Peptide stimulation and detection of intracellular IFN-γ in this example was carried out as in Example 1. Moreover, the "inflationary" T cells displayed a phenotype indicative of repeated activation 20 weeks after infection (CD127^{lo}, KLRG-1^{hi}), which is similar to T cells driven by wild-type MCMV (Figure 5C). "Inflationary" T cells express a similar phenotype throughout the chronic phase of infection (>3 months). This result indicated that, although unable to replicate, this "dead" virus continued to express viral proteins for at least 6 months.

It is therefore possible, that upon being introduced into the mouse some ΔgL MCMV gains access to CD34⁺ HSCs, which maintain and support the virus genome. The virus infects only a tiny percent of HSCs and cannot spread, and does not impair overall hematogenesis. However, it is likely that it is maintained in the body, undergoes abortive reactivation as the "infected" HSCs differentiate into DCs, and in the process stimulates immune responses.

### Example 3: Lack of T cell memory inflation in response to subcutaneous ΔgL MCMV infection

As shown in Example 2, CD8⁺ T cell memory inflation was driven by intraperitoneal infection of mice with wildtype and ΔgL MCMV. It is believed that the ability of ΔgL MCMV to elicit long lived self priming T cell memory is an outcome of the establishment of CMV latency in HSCs. Thus, any administration of CMV that allows access of virus to HSCs (as in intravenous administration) will likewise elicit characteristic T cell memory inflation. This example shows a failure to detect T cell memory inflation after subcutaneous administration of replication deficient MCMV.

### Methods

Mice were infected as in Examples 1 and 2. In this example however, mice were anesthetized with isofluorane and infected with wild-type or ΔgL MCMV by a single injection into the left-hind foot pad. The T cell response to both wild-type and ΔgL MCMV was measured from the peripheral blood by tetramer staining over time as in Example 2.

### Results

### The CD8⁺ T cell response to replication deficient ΔgL MCMV does not inflate after subcutaneous infection in the foot pad of the mouse.

The ability of replication-deficient MCMV to elicit memory "inflation" after subcutaneous infection was measured in C57BL/6 mice. As shown in Figure 6, antigen-specific CD8⁺ T cells inflate readily in mice infected with wild-type MCMV via the subcutaneous route. Filled circles represent wild-type infected mice and each line shows an individual mouse. Strikingly however, despite priming an acute CD8⁺ T cell response (M38-specific CD8⁺ T cells are evident at day 7 post infection), subcutaneous infection with the ΔgL virus failed to elicit memory "inflation". This result is consistent with the idea that ΔgL MCMV must gain access to a specific cell type like HSCs, in order to persist and drive T cell memory "inflation". Thus, these data suggest that the HSC target is not available in the foot pad site of injection. It is therefore currently believed that systemic infection (i.p. or i.v.) will be needed to drive T cell memory "inflation" using a replication-deficient CMV as a vector.

### Example 4: Failure of replication deficient MCMV to grow in severe combined immunodeficient animals

Using replication competent virus as a vaccine vector elicits substantial safety concerns. If the vaccinated individuals are or become immunosuppressed, viral replication and spread within that person could lead to disease and morbidity. Moreover, any vaccine that can spread to unvaccinated people in an uncontrolled setting could have disastrous consequences, especially if that individual is immunocompromised. However, a replication deficient vector alleviates those concerns because it can not spread either between individuals or within the vaccinated person. This example demonstrates that the ΔgL MCMV is truly replication deficient using mice with Severe Combined Immunodeficiency (SCID). These mice have no B or T cells and are thus extremely sensitive to viral infections.

### Methods

SCID mice were infected with different doses of wild-type MCMV or replication deficient ΔgL MCMV via a single intraperitoneal injection. The virus was allowed to grow in groups of animals until any mouse in that group demonstrated morbidity. Thus, in the example shown, after 36 days several mice infected with 10 PFUs of wild-type virus exhibited signs of morbidity (hunched posture, ruffled fur, swollen eyes, and sluggishness) and all mice in that group were sacrificed. Mice infected with ΔgL virus never exhibited signs of morbidity and were sacrificed 42 days after infection. Spleens, salivary glands, livers and lungs were harvested from all animals at the time of sacrifice and frozen with liquid nitrogen. Infected tissue was subsequently thawed, suspended in 1 ml of fresh T cell media (see Example 1 methods) and disrupted by Dounce homogenization using a drill. Aliquots of disrupted tissue were subsequently frozen at -80°C. DNA was harvested from a single aliquot of tissue using the QiaAmp kit from Qiagen. To measure the quantity of MCMV DNA present in the sample, quantitative PCR for the MCMV E1 gene was performed using the TaqMan® Universal Mastermix (Applied Biosystems). The primers and probe were synthesized by Invitrogen and Applied Biosystems respectively. VIC™ fluorescent reporter dye labeled probe: ACTCGAGTCGGACGCTGCATCAGAAT; Primer E1 for: TCGCCCATCGTTTCGAGA; Primer E1 rev: TCTCGTAGGTCCACTGACGGA. To calculate the genome copy number, a standard curve was included in every assay. For the standard curve, plasmid containing the E1 gene from MCMV (pGEM-T-E1) was titrated through 10-fold dilutions from 3x10⁵ copies to 3 copies per tube. Data was acquired on an Applied Biosystems, 7700 qPCR machine. Shown are data from the spleen of infected mice.

### Results

### Replication deficient MCMV fails to grow in Severe Combined Immunodeficient (SCID) mice.

The amount of virus present in the spleens of infected mice is shown in Figure 7. The data is represented as copy number of viral DNA per microgram of total DNA, which indicates the amount of virus in the infected organ. Each symbol represents an individual mouse. Filled circles represent animals infected with wild-type MCMV. When these mice displayed clear evidence of morbidity including a hunched posture, ruffled fur, swollen eyes, and sluggishness, the experiment was halted and tissue was harvested. It is clear from this data that as little as 10 pfu of wild-type MCMV can grow unchecked in SCID mice, which have no B cells or T cells. These mice began exhibiting signs of morbidity and had huge amounts of virus in their spleens 36 days after infection. However, mice infected with 100,000 pfu of the ΔgL virus never exhibited morbidity and were sacrificed 42 days after infection. At this time, no virus was detectible in the spleens of ΔgL infected animals (open circles, n=10). Thus, the infectious dose of replication deficient ΔgL used in these experiments is 10,000 x the amount of wild-type virus (10 pfu) needed to overwhelm SCID mice. The shaded area represents the limit of detection in these experiments. Spleens from uninfected mice were included for comparison (rightmost side of the graph). These data show that replication deficient virus fails to grow even in severe combined immunodeficient mice.

### Example 5: T cell memory inflation in response to intravenous ΔgL MCMV infection

This example discusses detection of T cell memory inflation by intravenously administered CMV.

The T cell response in mice infected intravenously with wildtype and ΔgL MCMV will be measured as described in Example 2. Mice will be infected by intravenous injections of wild type and ΔgL MCMV. Animals will also be infected subcutaneously and intraperitoneally as controls. Peripheral blood will be sampled prior to and at progressively following infection, and T cell responses to CMV peptide administration will be measured by tetramer staining as described.

### Example 6: Anti-tumor efficacy of CTL elicited by a replication-deficient CMV-vectored antigen

Example 1 demonstrates that a CMV-vectored antigen will provoke an effective immune response against a cell expressing that antigen. Further, as shown in example 2, CMV is able to repeatedly stimulate a characteristic CD8⁺T cell response regardless of the ability of the virus to spread between cells. It follows therefore that a replication-deficient CMV that is expressing a tumor antigen will likewise elicit a CD8⁺ T cell response that will effectively monitor the presence of and destroy tumor cells expressing the particular viral-vectored antigen.

This example describes methods of eliciting a long-term self priming immune response in a subject that is reactive to a particular cancer antigen. Also described are methods of treating a subject who has been diagnosed with a cancer, alone or in combination with chemotherapeutic, immunologic or radiation-based therapies.

### Replication-deficient CMV anti-tumor immunostimulating preparation in mice

MCMV lacking gL will be generated to express tumor antigens including, but not limited to, trp-2 and HER-2/neu. Mice will be injected i.p. or i.v. with these preparations, and several months later will be challenged with tumors, for example B16 melanoma cells for the trp-2 vaccine, and TUBO breast cancer cells for the HER-2/neu vaccine. The development of tumors will be compared with control mice as in Example 1.

Alternatively, MCMV lacking gL but expressing ovalbumin or HER-2/neu will be used to vaccinate transgenic mice (B6.FVB-Tg(MMTV-neu/OT-I/OT-II)CBnel Tg(Trp53R172H)8512Jmr/J) that spontaneously develop breast tumors expressing ova and HER-2/neu, commercially available from Jackson laboratories *(see* online at jaxmice.jax.org/strain/007962.html). In these mice, both ova and HER-2/neu are genuine self antigens. The mice will be monitored for development of tumors, compared to control mice.

Another model of spontaneous tumor development is HER-2/neu transgenic BALB/c mice. These mice will be infected with MCMV lacking gL expressing HER-2/neu, and monitored for spontaneous development of breast tumors.

### Replication-deficient CMV anti-tumor immunostimulating preparation in humans

Human CMV lacking gL can be developed expressing one or more humor tumor antigens, such as HER-2/neu, MAGE-1, MAGE-A3, trp-1, trp-2, gp100, CEA, MUC-1, PSA, or other cancer antigens. The resultant replication-deficient CMV viral vector preparation can be administered initially in phase I clinical trials to patients, for instance, who have failed conventional chemotherapy and/or radiotherapy treatments and who have advanced disease with a poor prognosis. One example would be a patient with stage 4 melanoma.

By way of example, the viral vector preparation can be administered intravenously. Patients are then monitored for indications of the viral vector having one or more biological effects, such as for instance: (a) development of T cells specific for the cancer antigen in their peripheral blood, (b) change in tumor mass, by imaging technique such as MRI, and (c) side effects of the vaccine, for instance by clinical and laboratory evaluation such as blood, kidney and renal function tests.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims.

## Claims

1. A recombinant, replication-deficient cytomegalovirus comprising a heterologous nucleic acid encoding a cancer antigen, for use in a method of generating a long term, repeatedly stimulated immune response against the cancer antigen in a subject, the method comprising:
administering to the subject, by intraperitoneal or intravenous administration, the recombinant, replication-deficient cytomegalovirus comprising a heterologous nucleic acid encoding the cancer antigen, whereby viral latency is established in the subject, thereby generating the repeatedly stimulated immune response against the cancer antigen.

2. The recombinant, replication-deficient cytomegalovirus for use according to claim 1, wherein the recombinant replication-deficient cytomegalovirus comprises an inactivated gL, gB, gD or gH glycoprotein gene.

3. The recombinant, replication-deficient cytomegalovirus for use according to claim 2, wherein the inactivated gene encodes the gL glycoprotein.

4. The recombinant, replication-deficient cytomegalovirus for use according to claim 4, wherein the gene encoding the gL glycoprotein is inactivated by a knock out mutation.

5. The recombinant, replication-deficient cytomegalovirus for use according to any of claims 1-4, wherein the nucleic acid encoding the cancer antigen is operably linked to a constitutive promoter.

6. The recombinant, replication-deficient cytomegalovirus for use according to any of claims 1-4, wherein the nucleic acid encoding the cancer antigen is operably linked to an inducible promoter.

7. The recombinant, replication-deficient cytomegalovirus for use according to any one of claims 1 to 4, wherein the subject has been diagnosed with a cancer, the cancer antigen is derived from the cancer, and the repeatedly stimulated immune response is immunity against the cancer, wherein the method also comprises administering to the subject one or a combination of a chemotherapeutic or a immunologic anti-cancer agent.

8. The recombinant, replication-deficient cytomegalovirus for use according to claim 7, wherein the method further comprises administering radiation therapy to the subject.

9. The recombinant, replication-deficient cytomegalovirus for use according to any of claims 1-8, wherein the recombinant replication-deficient cytomegalovirus is a human cytomegalovirus.

10. The recombinant, replication-deficient cytomegalovirus for use according to claim 9, wherein the human cytomegalovirus is selected from the group consisting of AD169, Davis, Toledo, and Towne.

11. The recombinant, replication-deficient cytomegalovirus for use according to any of claims 1-6, wherein the recombinant replication-deficient cytomegalovirus is a murine cytomegalovirus.

## Patentansprüche

1. Rekombinantes, replikationsdefizientes Zytomegalievirus, das eine heterologe Nucleinsäure umfasst, die für ein Krebsantigen kodiert, zur Verwendung in einem Verfahren zur Erzeugung einer langfristigen, wiederholt stimulierten Immunantwort gegen das Krebsantigen in einem Individuum, wobei das Verfahren Folgendes umfasst:
Verabreichen des rekombinanten, replikationsdefizienten Zytomegalievirus, das eine heterologe Nucleinsäure umfasst, die für das Krebsantigen kodiert, an das Individuum mittels intraperitonealer oder intravenöser Verabreichung, wodurch Viruslatenz im Individuum erzeugt wird, wodurch die wiederholt stimulierte Immunantwort gegen das Krebsantigen erzeugt wird.

2. Rekombinantes, replikationsdefizientes Zytomegalievirus zur Verwendung nach Anspruch 1, wobei das rekombinante, replikationsdefiziente Zytomegalievirus ein inaktiviertes gL-, gB-, gD- oder gH-Glykoproteingen umfasst.

3. Rekombinantes, replikationsdefizientes Zytomegalievirus zur Verwendung nach Anspruch 2, wobei das inaktivierte Gen für das gL-Glykoprotein kodiert.

4. Rekombinantes, replikationsdefizientes Zytomegalievirus zur Verwendung nach Anspruch 4, wobei das Gen, das für das gL-Glykoprotein kodiert, durch Knockout-Mutation inaktiviert ist.

5. Rekombinantes, replikationsdefizientes Zytomegalievirus zur Verwendung nach einem der Ansprüche 1-4, wobei die Nucleinsäure, die für das Krebsantigen kodiert, operabel an einen konstitutiven Promotor gebunden ist.

6. Rekombinantes, replikationsdefizientes Zytomegalievirus zur Verwendung nach einem der Ansprüche 1-4, wobei die Nucleinsäure, die für das Krebsantigen kodiert, operabel an einen induzierbaren Promotor gebunden ist.

7. Rekombinantes, replikationsdefizientes Zytomegalievirus zur Verwendung nach einem der Ansprüche 1 bis 4, wobei beim Individuum Krebs diagnostiziert wurde, das Krebsantigen vom Krebs stammt und die wiederholt stimulierte Immunantwort Immunität gegen den Krebs ist, wobei das Verfahren außerdem das Verabreichen eines oder einer Kombination eines Chemotherapeutikums oder eines immunologischen Antikrebsmittels an das Individuum umfasst.

8. Rekombinantes, replikationsdefizientes Zytomegalievirus zur Verwendung nach Anspruch 7, wobei das Verfahren weiters das Verabreichen von Strahlentherapie an das Individuum umfasst.

9. Rekombinantes, replikationsdefizientes Zytomegalievirus zur Verwendung nach einem der Ansprüche 1-8, wobei das rekombinante, replikationsdefiziente Zytomegalievirus ein menschliches Zytomegalievirus ist.

10. Rekombinantes, replikationsdefizientes Zytomegalievirus zur Verwendung nach Anspruch 9, wobei das menschliche Zytomegalievirus aus der aus AD169, Davis, Toledo und Towne bestehenden Gruppe ausgewählt ist.

11. Rekombinantes, replikationsdefizientes Zytomegalievirus zur Verwendung nach einem der Ansprüche 1-6, wobei das rekombinante, replikationsdefiziente Zytomegalievirus ein Maus-Zytomegalievirus ist.

## Revendications

1. Cytomégalovirus recombinant, à réplication déficiente, comprenant un acide nucléique hétérologue codant un antigène de cancer, pour utilisation dans une méthode de génération d'une réponse immune de longue durée, stimulée à répétition, contre l'antigène de cancer chez un sujet, la méthode comprenant :
administrer au sujet, par administration intrapéritonéale ou intraveineuse, le cytomégalovirus recombinant, à réplication déficiente, comprenant un acide nucléique hétérologue codant l'antigène de cancer, moyennant quoi une latence virale est établie chez le sujet, en générant ainsi la réponse immune stimulée à répétition contre l'antigène de cancer.

2. Cytomégalovirus recombinant, à réplication déficiente pour utilisation selon la revendication 1, où le cytomégalovirus recombinant à réplication déficiente comprend un gène de glycoprotéine gL, gB, gD ou gH inactivé.

3. Cytomégalovirus recombinant, à réplication déficiente pour utilisation selon la revendication 2, où le gène inactivé code la glycoprotéine gL.

4. Cytomégalovirus recombinant, à réplication déficiente pour utilisation selon la revendication 4, où le gène codant la glycoprotéine gL est inactivé par une mutation de décharge.

5. Cytomégalovirus recombinant, à réplication déficiente pour utilisation selon l'une quelconque des revendications 1 à 4, où l'acide nucléique codant l'antigène de cancer est fonctionnellement lié à un promoteur constitutif.

6. Cytomégalovirus recombinant, à réplication déficiente pour utilisation selon l'une quelconque des revendications 1 à 4, où l'acide nucléique codant l'antigène de cancer est fonctionnellement lié à un promoteur inductible.

7. Cytomégalovirus recombinant, à réplication déficiente pour utilisation selon l'une quelconque des revendications 1 à 4, où le sujet a été diagnostiqué comme étant atteint d'un cancer, l'antigène de cancer est dérivé du cancer, et la réponse immune stimulée à répétition est l'immunité contre le cancer, où la méthode comprend également l'administration au sujet d'un ou d'une combinaison d'un agent anti-cancer chimio-thérapeutique ou immunologique.

8. Cytomégalovirus recombinant, à réplication déficiente pour utilisation selon la revendication 7, où le procédé comprend en outre l'administration d'une radiothérapie au sujet.

9. Cytomégalovirus recombinant, à réplication déficiente pour utilisation selon l'une quelconque des revendications 1 à 8, où le cytomégalovirus recombinant, à réplication déficiente est un cytomégalovirus humain.

10. Cytomégalovirus recombinant, à réplication déficiente pour utilisation selon la revendication 9, où le cytomégalovirus humain est sélectionné dans le groupe consistant en AD169, Davis, Toledo et Towne.

11. Cytomégalovirus recombinant, à réplication déficiente pour utilisation selon l'une quelconque des revendications 1 à 6, où le cytomégalovirus recombinant, à réplication déficiente est un cytomégalovirus murin.


### SEQUENCE LISTING

<110> Oregon Health & Science University Hill, Ann B Snyder, Christopher M
<120> CYTOMEGALOVIRUS-BASED IMMUNOGENIC PREPARATIONS
<130> 899-83832-01
<160> 3
<170> PatentIn version 3.5
<210> 1
   <211> 26
   <212> DNA
   <213> Artificial Sequence
<220>
   <223> Synthetic probe used with fluorescent reporter dye label for detection of MCMV E1
<400> 1
   actcgagtcg gacgctgcat cagaat 26
<210> 2
   <211> 18
   <212> DNA
   <213> Artificial sequence
<220>
   <223> Forward primer used to amplify MCMV E1
<400> 2
   tcgcccatcg tttcgaga 18
<210> 3
   <211> 21
   <212> DNA
   <213> Artificial sequence
<220>
   <223> Reverse primer used to amplify MCMV E1
<400> 3
   tctcgtaggt ccactgacgg a 21